(19) Europäisches Patentamt
European Patent Office
Office européen des brevets

(11) EP 3 362 072 B1

(12) # EUROPEAN PATENT SPECIFICATION

(45) Date of publication and mention of the grant of the patent:
**10.05.2023 Bulletin 2023/19**

(21) Application number: **16855821.1**

(22) Date of filing: **14.10.2016**

(51) International Patent Classification (IPC):
***A61K 31/7032** (2006.01)* ***A61K 31/7048** (2006.01)*
***A61K 36/68** (2006.01)* ***A61P 3/02** (2006.01)*
***A61P 1/14** (2006.01)* ***A23K 10/30** (2016.01)*
***A23K 20/163** (2016.01)* ***A23K 20/10** (2016.01)*
***A23K 50/10** (2016.01)*

(52) Cooperative Patent Classification (CPC):
**A23K 10/30; A23K 20/10; A23K 20/163; A23K 50/10; A61K 31/7032; A61K 31/7048; A61K 36/68**

(86) International application number:
**PCT/NZ2016/050168**

(87) International publication number:
**WO 2017/065619 (20.04.2017 Gazette 2017/16)**

(54) **NITROGEN UTILISATION MODULATION**

STICKSTOFFNUTZUNGSMODULATION

MODULATION DE L'UTILISATION D'AZOTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.10.2015 NZ 15713302**

(43) Date of publication of application:
**22.08.2018 Bulletin 2018/34**

(73) Proprietor: **PGG Wrightson Seeds Limited**
**3204 Hamilton (NZ)**

(72) Inventors:
- **WESTWOOD, Charlotte**
  **3204 Hamilton (NZ)**
- **MOORHEAD, Allister**
  **3204 Hamilton (NZ)**
- **KEMP, Peter David**
  **3204 Hamilton (NZ)**
- **NAVARRETE QUIJADA, Soledad Del Carmen**
  **3204 Hamilton (NZ)**
- **JUDSON, Howard Glenn**
  **3204 Hamilton (NZ)**
- **STEWART, Alan**
  **3204 Hamilton (NZ)**

(74) Representative: **Boult Wade Tennant LLP**
**Salisbury Square House**
**8 Salisbury Square**
**London EC4Y 8AP (GB)**

(56) References cited:
- **ROB J. AERTS ET AL: "Polyphenols and agriculture: beneficial effects of proanthocyanidins in forages", AGRICULTURE, ECOSYSTEMS & ENVIRONMENT, vol. 75, no. 1-2, 1 August 1999 (1999-08-01), pages 1-12, XP055079349, ISSN: 0167-8809, DOI: 10.1016/S0167-8809(99)00062-6**
- **B. J. Malcolm ET AL: "The effect of four different pasture species compositions on nitrate leaching losses under high N loading", Soil Use and Management, vol. 30, n. 1, 17 February 2014 (2014-02-17), pages 58-68, XP055526854, DOI: 10.1111/sum.12101 Retrieved from the Internet: URL:https://api.wiley.com/onlinelibrary/tdm/v1/articles/10.1111%2Fsum.12101 [retrieved on 2018-11-26]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- J. LUO ET AL: "Nitrous oxide emission factors for urine and dung from sheep fed either fresh forage rape (Brassica napus L.) or fresh perennial ryegrass (Lolium perenne L.)", ANIMAL, vol. 9, no. 03, 19 November 2014 (2014-11-19), pages 534-543, XP055378152, GB ISSN: 1751-7311, DOI: 10.1017/S1751731114002742
- ELIZA GAWEL ET AL: "Protein from Lucerne in animals supplement diet", INTERNATIONAL JOURNAL OF FOOD, AGRICULTURE & ENVIRONMENT, WFL, HELSINKI, vol. 12, no. 2, 1 January 2014 (2014-01-01), pages 314-319, XP009510113, ISSN: 1459-0255, DOI: 10.1234/4.2014.4499
- MARKO DIETZ ET AL: "Inhibitory effects of Plantago lanceolata L. on soil N mineralization", PLANT AND SOIL, vol. 368, no. 1-2, 21 November 2012 (2012-11-21), pages 445-458, XP055584299, NL ISSN: 0032-079X, DOI: 10.1007/s11104-012-1524-9
- WOOWARD S L: "Can diverse pasture mixtures reduce nitrogen losses?", PROCEEDINGS OF THE 5TH AUSTRALASIAN DAIRY SCIENCE SYMPOSIUM : NOVEMBER 13 - 15, 2012, MELBOURNE, AUSTRALIA, AUSTRALASIAN DAIRY SCIENCE SYMPOSIUM ; 5 (MELBOURNE), AU, 13 November 2012 (2012-11-13), pages 463-464, XP009510497, ISBN: 978-0-646-58966-4
- GR EDWARDS ET AL: "Milk production and urination behaviour of dairy cows grazing diverse and simple pastures", PROCEEDINGS OF THE NEW ZEALAND SOCIETY OF ANIMAL PRODUCTION VOL, vol. 75, 1 January 2015 (2015-01-01), pages 79-83, XP055381939,
- JUDSON, HG et al.: "Benefits and uses of plantain (Plantago lanceolata) cv. Ceres Tonic in livestock production systems in New South Wales.", Proceedings of the 26th Annual Conference of The Grassland Society of NSW, 2011, pages 151-152, XP055378150,
- STEWART, AV: "Plaintain (Plantago lanceolata) - a potential pasture species.", Proceedings of the New Zealand Grassland Association, vol. 58, 1996, pages 77-86, XP055378151,
- AERTS, RJ et al.: "Polyphenols and agriculture: beneficial effects of proanthocyanidins in forages.", A griculture, Ecosystems and Environment, vol. 75, 1999, pages 1-12, XP055079349,
- MALCOLM, BJ et al.: "The effect of four different pasture species compositions on nitrate leaching losses under high N loading.", Soil Use and Management, vol. 30, 2014, pages 58-68, XP055526854,
- LUO, J et al.: "Nitrous oxide emission factors for urine and dung from sheep fed either fresh forage rape (Brassica napus L.) or fresh perennial ryegrass (Lolium perenne L.", Animal, vol. 9, no. 3, 2015, pages 534-543, XP055378152,
- GAWEL, E et al.: "Protein from Lucerne in animals supplement diet.", Journal of Food, Agriculture & Environment, vol. 12, no. 2, 2014, pages 314-319, XP009510113,

## Description

### TECHNICAL FIELD

The invention relates to uses of secondary plant metabolites selected from acteoside and catalpol for modulating nitrogen utilisation in animals.

In particular the invention relates to use of acteoside or catalpol for reducing the amount of nitrogen excreted in the urine of an animal. This invention has particular application to dairy cows although this should not be seen as limiting.

### BACKGROUND

An inevitable consequence of systems that farm animals is the production of faeces and urine both of which contain nitrogen (N).

Farmed animals excrete approximately 75% of the nitrogen that they ingest in their urine (50%) and faeces (25%). The nitrogen contained in excreted urine is generally in the form of urea, which is readily broken down to provide ammonium by, for example, urease present in the soil. Nitrification of the ammonium initially produces nitrite, which undergoes further nitrification to produce nitrate. High nitrate can be problematic because it is not retained by soil particles and readily leaches into groundwater before it can be utilised for plant growth. In some cases, such as under anaerobic conditions, the nitrate can denitrify to produce nitrogen gas, nitrous oxide, nitric oxide and ammonia, which in gaseous forms can be released to the air.

The risk of leaching nitrogenous products (particularly nitrate) into groundwater is especially acute in porous soils, such as gravels and after heavy rainfall events or in continually wet soils where water moves through the profile taking nitrogen below the root zone, where it could otherwise be utilised for plant growth.

Nitrogen is an essential macronutrient for pastures and any loss of nitrogen from the farm system must be accounted for primarily through the application of fertiliser or legume-Rhizobia fixation.

Where the groundwater serves as a source of drinking water, excessive nitrate levels have been linked with the infant disease commonly known as "blue baby syndrome" (methemoglobinemia) - particularly in bottle-fed babies younger than 4 months of age.

There are a number of methods that have been adopted to decrease the amount of nitrogen which enters the groundwater in farming regions, including the use of specific plant species which readily uptake nitrogen (as ammonium and/or nitrate) from the soil. One problem with relying on this technique is that as animal stocking levels increase, the plants cannot utilise enough of the excreted nitrogen to prevent leaching into the groundwater.

Another method employs the use of pasture species that, themselves, contain reduced levels of nitrogen. The urine produced by animals that consume these pasture species has a reduced level of nitrogen. At present, however, very few viable low-nitrogen plant species are available.

Another method to decrease groundwater pollution is to use stand-off pads which are areas that the animals are herded into to further digest the consumed herbage. These pads may comprise a hard (such as concrete) or soft (such as woodchip) surface and the animal excrement is contained and may be collected and further directed into storage ponds. A significant problem associated with this widely-used approach is the cost of building the infrastructure needed to collect, store and distribute the excrement.

A still further method to decrease nitrate leaching which has received recent interest is the use a chemical nitrification inhibitor which is typically sprayed onto the pasture prior to animals being allowed to graze the area. Both dicyandiamide (DCD) and 3,4-dimethlpyrazole phosphate (DMPP) slow the conversion of ammonium to nitrite by *Nitrosomonas spp.* and therefore limit the amount of nitrate being produced. Despite no widely recognised standard for an acceptable level of DCD in milk, the widespread use of DCD has been curtailed by the discovery of low levels in milk products produced by cows grazed in areas in which DCD has been sprayed. Furthermore, with a growing trend to produce organic produce, the use of chemical nitrification inhibitors in the organic farming sector is becoming less popular.

Aerts et al. (1999) "Polyphenols and agriculture: beneficial effects of proanthocyanidins in forages", AGRICULTURE, ECOSYSTEMS & ENVIRONMENT, vol. 75, no. 1-2, pages 1-12, describes the use of proanthocyanidins as secondary plant metabolites.

Malcolm et al. (2014) "The effect of four different pasture species compositions on nitrate leaching losses under high N loading", Soil Use and Management, vol. 30, n. 1, pages 58-68, describes the use of plantain in a diverse pasture.

Luo et al (2014) "Nitrous oxide emission factors for urine and dung from sheep fed either fresh forage rape (Brassica napus L.) or fresh perennial ryegrass (Lolium perenne L.)", ANIMAL, vol. 9, no. 03, pages 534-543 seeks to determine the partitioning of dietary nitrogen (N) between urine and dung in the excreta from sheep fed forage brassica rape or ryegrass, and then to measure N2O emissions.

Gawel et al. (2014) "Protein from Lucerne in animals supplement diet", INTERNATIONAL JOURNAL OF FOOD, AGRICULTURE & ENVIRONMENT, WFL, HELSINKI, vol. 12, no. 2, pages 314-319, describes the use of lucerne

concentrate as a natural fodder additive.

Dietz et al. (2012) "Inhibitory effects of Plantago lanceolata L. on soil N mineralization", PLANT AND SOIL, vol. 368, no. 1-2, pages 445-458, seeks to measure the effects of ribwort plantain, Plantago lanceolata L., and its allelochemicals on soil N mineralization

Woodward (2012) "Can diverse pasture mixtures reduce nitrogen losses?", PROCEEDINGS OF THE 5TH AUSTRALASIAN DAIRY SCIENCE SYMPOSIUM : NOVEMBER 13 - 15, 2012, MELBOURNE, AUSTRALIA, AUSTRALASIAN DAIRY SCIENCE SYMPOSIUM ; 5 (MELBOURNE), AU, pages 463-464, and Edwards et al. (2015) "Milk production and urination behaviour of dairy cows grazing diverse and simple pastures", PROCEEDINGS OF THE NEW ZEALAND SOCIETY OF ANIMAL PRODUCTION VOL, vol. 75, pages 79-83, each compares effects of a ryegrass while clover pasture with or without the addition of chicory, plantain and lucerne to the pasture.

There is a need for a farm management practice which leads to a reduction in nutrient leaching, particularly nitrate leaching, which addresses one or more of the foregoing problems or at least provides the public with a useful choice.

## SUMMARY OF THE INVENTION

In one aspect the invention provides a use of a secondary plant metabolite to reduce the amount of nitrogen excreted in the urine of an animal, wherein the secondary plant metabolite is selected from acteoside and catalpol.

Provided herein in accordance with uses of the invention is animal feed including a secondary plant metabolite having a concentration of 0.1 mg/g to 100 mg/g on a dry matter basis.

The inventors have now determined that the use of secondary plant metabolites modulates the utilisation of nitrogenous inputs in animals, and so provides a useful tool to manipulate nitrogen partitioning between animal products and urine.

By modulating the proportion of ingested nitrogen that is excreted in the urine, it is possible to control the amount of nitrogen waste that leaches below the root zone. In this way, secondary plant metabolites may be used to mitigate nitrate leaching.

In particular it has been found that the concentration of nitrogen in the urine of an animal that is administered a secondary plant metabolite is reduced compared to an animal that is not administered a secondary plant metabolite (such as fed on ryegrass and/or clover). In this respect, in one aspect the invention provides a use of a secondary plant metabolite to reduce urinary nitrogen concentration in an animal, wherein the secondary plant metabolite is selected from acteoside and catalpol. By modulating the utilisation of nitrogenous inputs in animals, and thereby manipulating nitrogen partitioning between animal products and urine, secondary plant metabolites described herein may be used in reducing nitrogen loading in individual urine patches derived from an animal.

In a further aspect the invention provides a use of a secondary plant metabolite for reducing the amount of nitrate derived from an animal that is leached into groundwater and/or surface water, wherein the secondary plant metabolite is selected from acteoside and catalpol.

It will be understood that, consequentially, by reducing the amount of nitrate derived from an animal that is leached into groundwater and/or surface water, the nitrate levels in surface water and/or groundwater in the vicinity of the animal will trend towards levels that would exist in the absence of the animal. Accordingly, secondary plant metabolites described herein may be used to reduce nitrate levels in surface water and/or groundwater in the vicinity of an animal.

The present invention is particularly advantageous to be used in the vicinity of sensitive catchments and waterways, such as are found in New Zealand, but also elsewhere.

Under certain conditions, such as under anaerobic conditions, it is well understood that nitrate can denitrify to produce nitrogen gas, nitrous oxide, nitric oxide and/or ammonia, which can be released to the air. Having nitrogen as a common element, each of these gases may be referred to as nitrogenous gases, some of which can be environmentally harmful. In particular, nitrous oxide is a known greenhouse gas.

In a still further aspect the invention provides a use of a secondary plant metabolite to reduce nitrogenous gases derived from an animal, wherein the secondary plant metabolite is selected from acteoside and catalpol.

In yet another aspect the invention provides a use of a secondary plant metabolite to reduce nitrogen volatilisation from a pastoral system, wherein the secondary plant metabolite is selected from acteoside and catalpol.

In a still further aspect the invention provides a use of a secondary plant metabolite to reduce greenhouse gas emissions from a pastoral system, wherein the secondary plant metabolite is selected from acteoside and catalpol.

In one aspect the invention provides a use of a secondary plant metabolite to reduce nitrate leaching from a pastoral system, wherein the secondary plant metabolite is selected from acteoside and catalpol.

## BRIEF DESCRIPTION OF DRAWINGS

Further aspects of the present invention will become apparent from the following description which is given by way of example only and with reference to the accompanying drawings in which:

Figure 1 Figure 1 shows metabolite production as a function of time for varying secondary plant metabolite concentrations: (A) acetate production (mmol/g DM), (B) propionate production (mmol/g DM) and (C) total VFA production (mmol/g DM) over time in all the *in vitro* treatments: CH, chicory; CH+1 0au, chicory + 10 mg aucubin /g DM; CH+20au, chicory + 20 mg aucubin/g DM; CH+40ac, chicory + 40 mg acteoside/g DM; PL, plantain (containing endogenous levels of 7 mg aucubin/g DM and 36 mg acteoside/g DM); PL+10au, plantain + an additional 10 mg aucubin/g DM; PL+36ac, plantain + an additional 36 mg acteoside/g DM. Bars denoting standard error of the mean at each time point are included at the top of each figure;

Figure 2 Figure 2 shows metabolite production as a function of time for varying secondary plant metabolite concentrations: (A) Butyrate production (mmol/g DM) and (B) branched VFA (isobutyrate and isovalerate) production (mmol/g DM) over time in all the *in vitro* treatments: CH, chicory; CH+10au, chicory plus 10 mg aucubin /g DM; CH+20au, chicory plus 20 mg aucubin/g DM; CH+40ac, chicory plus 40 mg acteoside/g DM; PL, plantain (containing endogenous levels of 7 mg aucubin/g DM and 36 mg acteoside/g DM); PL+10au, plantain plus an additional 10 mg aucubin/g DM; PL+36ac, plantain plus an additional 36 mg acteoside/g DM. Bars denoting standard error of the mean at each time point are included at the top of each figure;

Figure 3 Figure 3 shows net ammonia (mmol $NH_3$/g DM) production as a function of time for varying secondary plant metabolite concentrations for: (A) chicory *in vitro* treatments: CH, chicory; CH+10au, chicory plus 10 mg aucubin /g DM; CH+20au, chicory plus 20 mg aucubin/g DM; CH+40ac, chicory plus 40 mg acteoside/g substrate DM, and (B) plantain *in vitro* treatments: PL, plantain (containing endogenous levels of 7 mg aucubin/g DM and 36 mg acteoside/g DM); PL+10au, plantain plus an additional 10 mg aucubin/g DM; PL+36ac, plantain plus an additional 36 mg acteoside/g DM. Bars denoting standard error of the mean at each time point are included at the top of each figure.

## DETAILED DESCRIPTION OF THE INVENTION

Secondary plant metabolites are organic compounds that are not directly involved in the normal growth, development or reproduction of the plant.

In the uses disclosed and claimed herein, the secondary plant metabolite is selected from acteoside and catalpol. For example, acteoside contains a caffeic acid (phenylpropanoid) moiety and hydroxytyrosol (phenylethanoid) moiety.

In other examples not falling within the scope of the claims, the secondary plant metabolite may be any other glycoside. The glycoside may be a terpenoid glycoside, such as an iridoid glycoside, for example an iridoid glucoside. The glycoside may be a phenylpropanoid or phenylethanoid glycoside such as cistanoside F, lavandulifolioside, plantamajoside and isoacteoside.

Variants (including aglycones and stereoisomers) and derivatives of glycoside secondary plant metabolites are also contemplated. In this respect the aglycones of catalpol and acteoside may be used. Secondary plant metabolites for use in the present invention in accordance with the claims are acteoside, and catalpol, most particularly acteoside. Another secondary plant metabolite described herein is aucubin.

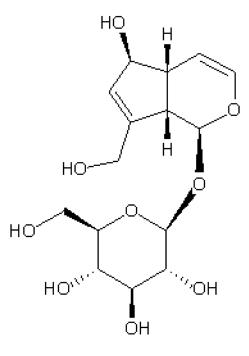

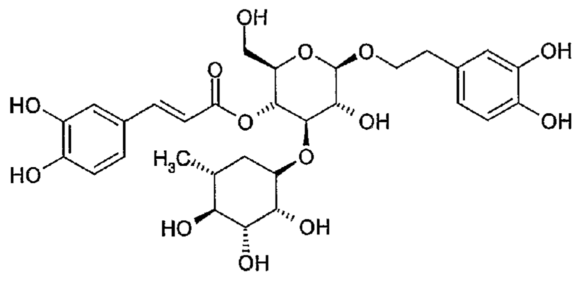

aucubin acteoside

Secondary plant metabolites themselves may provide an unappealing taste to an animal or may even be toxic

to the animal.

The inventors have determined that the administration of a particular amount of secondary plant metabolite, in preferred embodiments, provides two key requirements of the present invention, namely

a) reducing the proportion of total nitrogen ingested by an animal that is excreted in the urine of the animal, and doing this

b) without substantially affecting palatability and consequently voluntary feed intake of animals consuming the feed, or producing a substantial toxic effect.

As used herein the expression "administering the secondary plant metabolite to an animal" typically refers to any method of administering the secondary plant metabolite to the animal orally, typically ingesting the secondary plant metabolite so that is enters the rumen in those cases where the animal is a ruminant. In its simplest form the secondary plant metabolite may be administered in conjunction with the plant in which it was formed. In an exemplary embodiment, the secondary plant metabolite may be administered as part of the animal feed.

When administered as part of the animal feed, the feed is generally of plant origin. For example, the feed may be at least part of a forage crop that is eaten by grazing livestock as pasture or as crop residue (such as the residue left after a crop is harvested).

The feed may be at least part of a forage crop that is eaten by livestock after having been conditioned into, for example, balage or conserved feed including, but not limited to, silage, balage, hay or haylage.

In such cases where the feed is of plant origin, the plant material itself may have produced the secondary plant metabolite and thereby incorporate secondary plant metabolite within its structure.

Advantageously, particularly where the plant material provides the source of the secondary plant metabolite, the present invention provides the user with a use of secondary plant metabolites for readily providing a feeding animal with nutrition whilst simultaneously reducing the deleterious environmental impact of the animal. At its simplest, the use of secondary plant metabolites of the invention is able to be performed by grazing an animal on a pasture which includes a plant material including a secondary plant metabolite.

When administered as part of the feed the secondary plant metabolite may preferably be present at a concentration of 0.1 to 100 mg/g (on a dry matter basis), such as 0.5 mg/g to 70 mg/g.

It has been discovered that the use of aucubin at a concentration of 0.1 to 100 mg/g of dry matter, such as 0.1 mg/g to 45 mg/g (dry matter) has a significant effect on reducing the proportion of total nitrogen that is ingested by an animal that is excreted in the urine of the animal. This concentration also does not cause a significant reduction in food weight consumed by the animal due to poor taste, nor any noticeable toxic effects.

For example, aucubin has been effective when administered at a concentration of 0.1 mg/g to 45 mg/g (dry matter), such as at a concentration of 25 to 40 mg/g (dry matter). For example, aucubin may be administered as part of a feed that includes a plant species (such as plantain) having a concentration of 0.1 mg/g to 45 mg/g (dry matter), such as a concentration of 25 to 40 mg/g (dry matter). In some instances, the total feed may include 10-25% of a plant species (such as plantain) having a concentration of 0.1 mg/g to 45 mg/g (dry matter).

Without wishing to be bound by theory, these exemplary concentration ranges provide a reliable effect on nitrogen partitioning accounting for seasonal fluctuations in concentrations of aucubin within the plant and also allow a limited proportion of plantain to be present in the diet whilst still providing an effect on nitrogen partitioning.

The inventors have also determined that the use of acteoside at a concentration of 0.1 to 100 mg/g of dry matter, such as 0.5 mg/g to 70 mg/g (dry matter) has a significant effect on of reducing the proportion of total nitrogen ingested by an animal that is excreted in the urine of the animal. This concentration also does not cause a significant reduction in food weight consumed by the animal due to poor taste, nor any noticeable toxic effects.

For example, acteoside may be administered at a concentration of 0.5 mg/g to 70 mg/g (on a dry matter basis), such as at a concentration of 25 to 45 mg/g (on a dry matter basis). For example, acteoside may be administered as part of a feed that includes a plant species (such as plantain) having a concentration of 0.5 mg/g to 70 mg/g (dry matter), such as a concentration of 25 to 45 mg/g (dry matter). In some instances, the total feed may include 10-25% of a plant species (such as plantain) having a concentration of 0.5 mg/g to 70 mg/g (dry matter).

Without wishing to be bound by theory, these exemplary concentration ranges provide a reliable effect on nitrogen partitioning accounting for seasonal fluctuations in concentrations of acteoside within the plant and also allow a limited proportion of plantain to be present in the diet whilst still providing an effect on nitrogen partitioning.

Some plant species used as a forage crop naturally contain secondary plant metabolites suitable for use in the present invention, although most do not.

Exemplary species that naturally contain secondary plant metabolites are of the genus *Plantago,* such as *Plantago lanceolata,* known as plantain, which is often thought of as a weed. In many countries, plantain is not used as a forage crop, and is even eradicated from pasture systems.

Preferably a plantain having a Mediterranean genetic origin is used. Preferably the plantain provides winter growth. A preferred example of a plantain is *Plantago lanceolata.* A particularly preferred example of a plantain is *Plantago lanceolata* cv. Ceres Tonic, and breeding lines largely derived from that breeding pool. One such example of a breeding line is PG742.

In a preferred embodiment in accordance with the uses of the invention, the animal feed including a secondary plant metabolite having a concentration of 0.1 mg/g to 100 mg/g on a dry matter basis includes a species of the genus *Plantago.*

In a further preferred embodiment in accordance with the uses of the invention, the animal feed including a secondary plant metabolite having a concentration of 0.1 mg/g to 100 mg/g on a dry matter basis includes feed derived from plants of the species *Plantago lanceolata.* By way of example, the animal feed may include feed derived from plants of the species *Plantago lanceolata* as part of a combination of feeds, including a combination of plant-based feeds, or may include feed derived from plants of the species *Plantago lanceolata* alone.

In some embodiments in accordance with the uses of the invention the animal feed is derived from a pasture system which includes at least one plant of the genus *Plantago,* and optionally also includes at least one plant selected from one or more of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*).

In some embodiments in accordance with the uses of the invention the animal feed is derived from a pasture system which includes at least one plant of the genus *Plantago,* and optionally also includes at least one plant selected from between only one and four of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*).

In some embodiments in accordance with the uses of the invention the animal feed is derived from a pasture system which includes at least one plant of the genus *Plantago,* and optionally also includes at least one plant selected from between only one and three of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*).

In some embodiments in accordance with the uses of the invention the animal feed is derived from a pasture system which includes at least one plant of the genus *Plantago,* and optionally also includes at least one plant selected from only one or two of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*).

In some embodiments in accordance with the uses of the invention the animal feed is derived from a pasture system which includes at least one plant of the genus *Plantago,* and optionally also includes at least one plant selected from only one of the following: ryegrass (*Lolium* spp.), clover (*Trifolium* spp.), cocksfoot (*Dactylis glomerata*), chicory (*Cichorium intybus*), tall fescue (*Festuca arundinacea*), Kentucky bluegrass (*Poa pratensis*), birdsfoot trefoil (*Lotus corniculatus*), lucerne (*Medicago sativa*), and prairie grass (*Bromus willdenowii*).

Advantageously, plants of the genus *Plantago* allow for similar productivity to more diverse pasture systems, such that plants of the genus *Plantago* can be used as the predominant or even sole animal food source (Judson, H.G., McAnulty, R. and Sedcole, R. Proceedings of the New Zealand Grassland Association 71: 201-205 (2009); and Box, L.A., Edwards, G.R. and Bryant, R.H. Proceedings of the New Zealand Society of Animal Production 2016. Vol 76: 18-21). It has been disclosed that "Addition of plantain to pasture mixes provided significant dry matter production advantages which ranged from 6t DM/ha in the first year to 1.2t DM/ha by Year 3" (Moorhead, A.J.E. and Piggot, G.J. Proceedings of the New Zealand Grassland Association 71: 195-199 (2009)).

In an example not falling within the scope of the claims, there is described a method of reducing the proportion of total nitrogen ingested by an animal that is excreted in the urine of the animal, the method including the step of administering aucubin to the animal. In certain examples the aucubin is administered at a concentration of 0.1 mg/g to 45 mg/g (dry matter), such as at a concentration of 25 to 40 mg/g (dry matter).

In a further example not falling within the scope of the claims is described the use of aucubin to reduce the proportion of total nitrogen ingested by an animal that is excreted in the urine of the animal. Aucubin may preferably be so used at a concentration of 0.1 mg/g to 45 mg/g (dry matter), such as at a concentration of 25 to 40 mg/g (dry matter).

In a further example not falling within the scope of the claims is described animal feed including aucubin having a concentration of 0.1 mg/g to 45 mg/g (dry matter), such as at a concentration of 25 to 40 mg/g (dry matter).

In a further example not falling within the scope of the claims is described a method of reducing the proportion of total nitrogen ingested by an animal that is excreted in the urine of the animal, the method including the step of administering acteoside to the animal.

The desired concentration of the secondary plant metabolite in the feed may be achieved by a number of different methods.

The feed may contain a plant that naturally produces the secondary plant metabolite. Such a plant may be selected from a population of plants of a given species for the desired concentration of secondary plant metabolite. The overall concentration of the secondary plant metabolite can then be adjusted down by effectively diluting the plant containing the secondary plant metabolite with one or more other plant species that produce less secondary plant metabolite or even zero secondary plant metabolite, or at least less of certain secondary plant metabolites (such as aucubin or acteoside).

The feed may contain a plant that has been engineered using recombinant DNA techniques, for example, to provide the secondary plant metabolite at the desired concentration.

Alternatively, or in combination with using a plant species that naturally contains the secondary plant metabolite, the feed may be fortified using a concentrate composition including the secondary plant metabolite. The concentrate composition may be a powder, pellet, paste, etc, wherein the desired concentration of secondary plant metabolite is achieved by the addition of a predetermined quantity of the composition including the secondary plant metabolite.

Secondary plant metabolite concentration may vary throughout a growing season.

For example, the concentration of aucubin and acteoside in plantain swards can increase throughout the growing season. The concentration of these compounds in plantain is believed to be influenced by genetic and environmental factors, as well as leaf age.

Plantain (cultivar Ceres Tonic) exhibits very low levels of catalpol - it being nearly absent of this iridoid glycoside. Catalpol is biologically synthesised from its precursor aucubin. On the other hand, the concentrations of aucubin and acteoside may increase similarly during the growing season and the highest concentration of both compounds is typically in autumn. High concentrations of aucubin of up to 30 mg/g (depending on the genotype) and from 10 to 27 mg/g (dry matter) for the cultivar Ceres Tonic have been reported. Whereas, acteoside concentrations as high as 60 to 90 mg/g (dry matter) have been reported in natural ecotypes and as low as 3.2 mg/g (dry matter) of acteoside in the plantain cultivar Ceres Tonic.

The secondary plant metabolite may also be administered to the animal as a separate dose which is not part of the animal feed.

Without wishing to be bound by theory, it is believed that the secondary plant metabolite acts by modulating bacterial populations in the rumen. Accordingly it is preferred that any separate dose of secondary plant metabolite is administered to the animal by a mode that allows the secondary plant metabolite to enter the rumen - typically by oral ingestion. Such orally ingestible forms are typically palatable to the animal to aid with compliance.

A number of the secondary plant metabolites contemplated by the present invention are relatively hydrophilic, and accordingly standard methods of formulating such compounds may be employed.

The secondary plant metabolites may be accepted voluntarily by the animal (such as in the form of a palatable edible dosage form including a bolus, including a modified release bolus, or a medicated block) or may require more substantial human intervention such as by drench (paste, solution, emulsion, suspension) or gavage (solid form).

Where the secondary plant metabolite is administered as a separate dose which is not part of the animal feed, preferably the dosage is calculated based on the amount of dry matter being otherwise consumed contemporaneously with the administration of the dose.

For example, if a lactating dairy cow is consuming the equivalent of 4% of her bodyweight in dry matter per day (for example 4% of 500 kg = 20 kg DM), then the dose of secondary plant metabolite to administer at a rate of 0.1 mg/g to 100 mg/g (dry matter basis) can be calculated as 2 g to 2000 g per day. For a 200 kg heifer eating 4% of 200 kg = 8 kg the corresponding dose range would be 0.8 g to 800 g per day.

The dosage may be provided as a single dose, or a plurality of doses throughout the day. The dosage may be provided for a single day, or over a plurality of days which may or may not be sequential. The dosage regime will be chosen based on the desired result, being the desired degree of reduction of the proportion of total nitrogen ingested by an animal that is excreted in the urine of the animal.

Dosage of the secondary plant metabolite may also be determined as a weight per liveweight of the animal being administered with the secondary plant metabolite.

For a 200 kg heifer, eating 2 kg of ensiled plant material (per day) which includes 0.1 mg/g of secondary plant metabolite, the dosing rate is 0.2 g per 200 kg liveweight, which corresponds to 1 mg/kg liveweight.

Likewise, 100 mg/g of secondary plant metabolite consumed by the same animal corresponds to about 1 g/kg liveweight.

It will be understood that providing the same supply of feed to a given population of animals may result in variance in the dosing relationship, proportional to the liveweights of the individual animals. For example, a 500 kg dairy cow eating 2 kg of silage at 0.1 mg/g has a dose rate of 0.4 mg/kg liveweight, which is less than half that of a smaller cow having a smaller liveweight.

In a preferred embodiment the secondary plant metabolite is administered at a rate of about 0.5 to 4000 mg/kg liveweight per day, such as 1-1000 mg/kg liveweight per day.

As used herein the term "reducing", "reduce", "reduction" (as the context requires) refers to a modulation of a

level (typically the level of nitrogen) by the application/administration of a secondary plant metabolite, so that the level changes from a first nitrogen level to a second nitrogen level. The first nitrogen level can, or could, be determined in the absence of applying/administering the secondary plant metabolite. The second nitrogen level can or could be determined in the presence of applying/administering the secondary plant metabolite.

The reduction of the proportion of total nitrogen ingested by an animal that is excreted in the urine of the animal can be determined using a number of different techniques.

Although nitrogen may be sourced from a water supply and ingested by the animal by that mode, typically the overwhelming majority of inputted nitrogen is ingested by an animal through the feed that is consumed. Calculating the amount of nitrogen that is consumed in the feed can generally be done without difficulty, and may make use of digestion techniques including the Kjeldahl method and by combustion using a Leco analyser, although the use of Near-IR testing is considered to be a robust testing method. Similarly, measuring the nitrogen content of the animal's urine is without difficulty and can make use of similar techniques.

For a single animal, the reduction of the proportion of total nitrogen ingested by an animal that is excreted in the urine of the animal can be calculated by first determining the proportion of ingested nitrogen that is excreted in the urine before administering the uses of the present invention to provide a nitrogen proportion before administration - a first nitrogen proportion (NP1).

Following administration of the uses of the present invention it will be possible to determine a nitrogen proportion after administration - a second nitrogen proportion (NP2). The present invention advantageously provides uses which result in:

$$\text{first nitrogen proportion (NP1)} > \text{second nitrogen proportion (NP2)}$$

The degree (as a percentage) by which the proportion of total nitrogen ingested by an animal that is excreted in the urine of the animal is reduced can be calculated as follows:

$$100 \times [NP1 - NP2]/NP1$$

For a population of animals, the reduction in the proportion of total nitrogen that is ingested by any given animal may be calculated as a statistical average based on a control sub-population within the population which is not subjected to the uses of the present invention.

The control sub-population may be provided with a feed with a similar nitrogen content to the test sub-population which consume the secondary plant metabolite(s), but which does not contain any significant level of the secondary plant metabolite(s) specifically being tested for in the test sub-population.

The degree (as a percentage) by which the proportion is reduced may be chosen by the skilled addressee based on the desired outcome.

The desired outcome may be to maximally reduce the proportion during periods of greatest soil moisture content to reduce leaching to the groundwater.

Alternatively, during drier periods the desired reduction may be minimal in order to continue to provide a source of nitrogenous fertiliser.

The reduction in the proportion of total nitrogen ingested by an animal that is excreted in the urine of the animal is typically greater than 1%, such as greater than 5%, greater than 10%, greater than 20%, greater than 30%, greater than 40% or greater than 50%.

It will be understood that typically the uses of the invention do not result in 100% removal of nitrogen from the urine. In general the reduction in the proportion of total nitrogen ingested by an animal that is excreted in the urine of the animal is less than 90%, such as less than 80%, less than 70%.

In an example, the reduction in the proportion of total nitrogen ingested by an animal that is excreted in the urine of the animal is between 10% and 90%, such as between 20% and 80%.

The present invention is particularly well suited to modulating the nitrogen utilisation of domesticated farm animals, including mammals. Examples of such mammals are cattle, horses, sheep, goats and pigs. Preferably the present invention is for use in modulating the nitrogen utilisation of ruminant animals (such as cattle, goats, sheep, yaks, deer) - particularly cattle and sheep. Still more preferably the present invention is for use in modulating the nitrogen utilisation of dairy cattle and sheep. Dairy cows are most preferred as they form the largest part of the dairy herd and are believed to contribute the most to nitrate leaching.

As used herein, the expression "in the vicinity of an animal", particularly with respect to reducing nitrate levels in surface water and/or groundwater, refers to the sphere of influence of the animal on nitrate levels in that environment. For example, it will be understood that the impact of the animal on nitrate levels in surface water and/or groundwater

will most noticeably be felt downstream (surface water) or down-gradient (groundwater) and that effect will typically decay as a function of distance from the animal. Nonetheless the effect may be observed some kilometres from the animal, however typically the sphere of influence will extend the vicinity of the animal to less than 5 km, such as less than 1 km, such as less than 100 m from the animal. Most noticeably the effect of the invention herein will be observed within 5 m of where the animal has passed in any given day in which the secondary plant metabolite is consumed.

Nonetheless, it will also be appreciated that where standoff pads are used to collect urine from animals, and where the urine is subsequently dispersed as liquid fertiliser, the sphere of influence of the animal may extend to those fertilised areas where the animal may not have passed for a significant period of time, if at all. In those cases, the reduced nitrate levels achieved in that fertilised land could still justifiably be said to have benefited from the invention and accordingly would fall within the expression "in the vicinity of an animal", since those fertilised areas are typically on the same broader farm/pastoral system that the animal has inhabited.

No admission is made that any reference constitutes prior art. The discussion of the references states what their authors assert, and the applicants reserve the right to challenge the accuracy and pertinency of the cited documents. It will be clearly understood that, although a number of prior art publications are referred to herein, this reference does not constitute an admission that any of these documents form part of the common general knowledge in the art, in New Zealand or in any other country.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

The invention may also be said broadly to consist in the parts, elements and features referred to or indicated in the specification of the application, individually or collectively, in any or all combinations of two or more of said parts, elements or features.

Where in the foregoing description reference has been made to integers or components having known equivalents thereof, those integers are herein incorporated as if individually set forth.

It should be noted that various changes and modifications to the presently preferred embodiments described herein will be apparent to those skilled in the art.

Aspects of the present invention have been described by way of example only and it should be appreciated that modifications and additions may be made thereto without departing from the scope thereof.

## EXAMPLES

### *In vitro* trials

Experimental:

Plant material

The plantain (*Plantago lanceolata* L cv Ceres Tonic) and chicory (*Cichorium intybus* L cv Grassland Choice) evaluated in this study were collected from pure swards established in October 2011 and managed with 2 grazing frequencies (2 weekly and 4 weekly) throughout two growing seasons (2011-2012 / 2012-2013). The plantain and chicory pastures were in a randomised complete block design with five replicates in a factorial arrangement of the pasture. Hand plucked samples from the pure plantain and pure chicory swards were taken in December 2011 and May 2012 (in the first growing season) and in October 2012, January 2013, and May 2013 (in the second growing season) to determine the concentration of catalpol, aucubin and acteoside.

Laboratory analysis of secondary plant metabolite

Plantain and chicory samples were stored at -20 °C until analysis, then freeze-dried and ground to pass through a 1 mm diameter sieve. Catalpol, aucubin, and acteoside concentration in plantain were determined by HPLC (high performance liquid chromatography).

*In vitro* experimental design.

The *in vitro* gas production technique was used to investigate the impact of aucubin (au) and acteoside (ac) (>90% and >99% pure, respectively; Extrasynthese S.A, France) on rumen fermentation characteristics at higher concentration to those we found in plantain. Chicory, a herb forage, that does not contain detectable levels of these compounds, was used as a negative control substrate and plantain, that naturally contains both compound, was used as positive control. The treatments evaluated were:(i) chicory negative control (CH), 0 mg aucubin or acteoside/g DM (CH);

(ii) chicory with 10 mg aucubin/g DM (CH+10au); (iii) chicory with 20 mg aucubin/g DM (CH+20au); (iv) chicory with 40 mg acteoside/g DM (CH+40ac); (v) plantain positive control (PL), with existing natural levels of aucubin and acteoside (7 mg/g DM and 36 mg/g DM respectively; Table 1); (vi) plantain with an additional 10 mg aucubin/g DM (PL+10au); and (vii) plantain with an additional 36 mg acteoside/g DM (PL+36ac). The treatments are further summarised in Table 1. The chicory and plantain used in this *in vitro* study as substrate were from the pastures grazed every 4 weeks and were harvested in May 2013.

**Table 1.** Summary of the Chicory (CH) and Plantain (PL) treatments and concentration of the aucubin (au) and acteoside (ac) tested.

| | | aucubin and acteoside concentration (mg/g DM) | | |
|---|---|---|---|---|
| Treatments | Substrate | natural level | level added | Total |
| CH | Chicory | 0 aucubin | 0 aucubin | 0 aucubin |
| | | 0 acteoside | 0 acteoside | 0 acteoside |
| CH+10au | Chicory | 0 aucubin | **10 aucubin** | 10 aucubin |
| | | 0 acteoside | 0 acteoside | 0 acteoside |
| CH+20au | Chicory | 0 aucubin | **20 aucubin** | 20 aucubin |
| | | 0 acteoside | 0 acteoside | 0 acteoside |
| CH+40ac | Chicory | 0 aucubin | 0 aucubin | 0 aucubin |
| | | 0 acteoside | **40 acteoside** | 40 acteoside |
| PL | Plantain | 7 aucubin | 0 aucubin | 7 aucubin |
| | | 36 acteoside | 0 acteoside | 36 acteoside |
| PL+10au | Plantain | 7 aucubin | **10 aucubin** | 17 aucubin |
| | | 36 acteoside | 0 acteoside | 36 acteoside |
| PL+36ac | Plantain | 7 aucubin | 0 aucubin | 7 aucubin |
| | | 36acteoside | **36 acteoside** | 72 acteoside |

*In vitro* incubations

The *in vitro* fermentation assay was undertaken over 24 h using an incubator with the capacity for 32 bottles (Contherm Scientific Limited). All treatments were incubated in two sets of duplicate bottles where one set of bottles was used to record gas production and the other set used to determine products of fermentation, $NH_3$ and VFA. The incubations were carried out on three occasions and evaluated three replicate herbage samples (derived from separate plots in the field, n=3).

Approximately 300 mg DM of substrate (1 mm ground plantain and chicory) was weighed into a 125 mL serum bottle. Aucubin and acteoside were then added to the chicory and plantain bottles at the concentrations outlined in Table 1.

Three fistulated cows were used as rumen fluid donors with rumen fluid obtained in the morning from multiple sites within the rumen and transferred to the laboratory in pre-warmed thermos flasks. Once in the laboratory the rumen fluid was strained through a double layer of cheesecloth, pooled in equal proportions, and mixed with McDougal's buffer in a 1:4 ratio, under continuous flushing with $CO_2$ in a water bath at 39°C to obtain rumen fluid as source of inoculum. Then 30 mL of this buffered rumen fluid was injected into each bottle containing substrate plus or minus additional secondary compounds and sealed with rubber stoppers, manually agitated and place inside the incubator at 39°C for 24 h.

Determination of gas production and end fermentation products

The headspace gas pressure inside each bottle was measured automatically using a pressure sensor connected to a needle in the cap of the incubation bottle (40PC015G1A, Honeywell, International Inc., Morris town, NJ, USA). Pressure was recorded every minute and when pressures exceeded 10 kPa the accumulated gas was released.

The set of bottles for $NH_3$ and VFA determination were repeat sampled at 1.5, 3, 5, 8, 12 and 24 hours taking 1.8 mL of medium from each bottle at each sampling time. These subsamples were transferred to micro tubes and centrifuged at 2100 x *g* for 10 min at 4° C. Then duplicate 0.9 mL aliquots of the supernatant were transferred into micro-tubes with 0.1 mL of an internal standard (19.87 mM ethyl butyric acid, 20% v/v ortho-phoric acid) and frozen at -20°C for $NH_3$ and VFA analysis.

Laboratory analysis for in vitro incubations

Plantain and chicory used as substrate were analysed for total dry matter (DM) which was determined by drying the samples at 105 °C in a conventional oven (AOAC, 2000; method 930.15, 925.10) and organic matter (OM) by heating the samples for 16 h at 550 °C in a furnace (AOAC, 2000; method 942.05). Total nitrogen (N) was determined by combustion using a Leco analyser (AOAC, 2000; method 968.06), and crude protein (CP) was calculated by multiplying the N content by 6.25. Neutral detergent fibre (NDF), acid detergent fibre (ADF) and lignin were analysed in a Tecator Fibretec System (Foss Fibretec, Höganäs, Sweden). Cellulose was calculated as ADF less lignin, and hemicellulose as NDF less ADF. Hot water soluble carbohydrates (HWSC) were measured by the reducing sugar method.

The samples for VFA and $NH_3$ analysis were thawed and centrifuged at 2100 × *g* for 10 min at 4° C, and 0.8 mL of the supernatant was transferred into a crimp cap vial to determine individual VFA (acetate, propionate, butyrate, isobutyrate, and isovalerate) by gas chromatography (HP 6890, Santa Clara, CA, USA). Part of the final supernatant (approx. 0.1 mL) was used to determine $NH_3$ concentration by a colorimetric method.

Calculations and statistical analysis

The gas production (GP) profiles of each bottle were fitted using the following formula

$$V = (A\ (1\text{-}\exp(\text{-}kt))/\ (1+\exp(\ln(1/d)\text{-}kt))$$

where:

V: total gas produced at time t (mL/g DM)

A: is the asymptotic potential of GP (mL/g DM)

k= is the fractional rate gas production per hour (/h)

d= is a shape parameter indicating a sigmoidal or no sigmoidal shape of the gas curve.

The half time of when the asymptotic was reached ($T^{1/2A}$) and the fermentation rate ($R^{1/2A}$) at $T^{1/2A}$ were calculated as follow:

$$T^{1/2A} = (\ln(2+1/d))/k$$

$$R^{1/2A} = (k(d+0.5))/(1+d)$$

The concentration of catalpol, aucubin, and acteoside in plantain, the model parameters (A, $T^{1/2A}$, $R^{1/2A}$ and V24h) and the fermentation end products after 24 hours of incubation were analysed with incubation day treated as a random effect. Net $NH_3$ production and individual VFA production over time were analysed using the PROC MIXED procedure in SAS in a design where treatments and time (1.5, 3, 5, 8, 12 and 24 h) and the interaction of treatment and time where fitted as fixed effects. The inclusion of incubation day as a random effect provided true replication. Means were compared using the least squares means test and significance was declared at $P < 0.05$.

Results:

Secondary compounds in plantain

The concentration of secondary compounds found naturally occurring in the plantain samples harvested from the two growing season and two grazing frequencies, are presented in Table 2. Catalpol was detected at very low concentrations, ranging from 0.01 to 0.06 mg/g DM, with no differences observed across the growing season harvest dates or due to grazing frequency. Aucubin and acteoside concentrations increased (P<0.05) over the growing season in both years (Table 2).

There was an interaction (P<0.05) between grazing frequency and harvest date observed for aucubin concentration in the first growing season only, whereby frequent grazing (2 weekly) appeared to inhibit aucubin accumulation

towards the end of the growing season (May). No effect of grazing frequency was observed in relation to acteoside concentration, and nor was any interaction observed during the second growing season.

**Table 2.** Concentration (mg/g DM) of catalpol, aucubin and acteoside in plantain (mean ± SEM; n=5)

| | catalpol | aucubin | acteoside |
|---|---|---|---|
| ***First growing season (Dec 2011 - May 2012)*** | | | |
| Grazing frequency | | | |
| 2 weekly | 0.03 ± 0.01 | 1.99 ± 0.29$^{b}$ | 25.71 ± 4.06 |
| 4 weekly | 0.02 ± 0.01 | 3.59±0.89$^{a}$ | 33.31 ± 4.17 |
| Date | | | |
| Dec-2011 | 0.03 ± 0.01 | 1.78± 0.24$^{b}$ | 23.61 ± 3.03$^{b}$ |
| May-2012 | 0.02 ± 0.01 | 3.80± 0.80$^{a}$ | 35.40± 3.96$^{a}$ |
| Grazing frequency * date | | | |
| 2weekly*Dec-2011 | 0.05 ± 0.02 | 1.80 ± 0.35$^{c}$ | 20.59 ± 3.36 |
| 4weekly*Dec-2011 | 0.02 ± 0.01 | 1.75 ± 0.10$^{c}$ | 26.63 ± 3.20 |
| 2weekly*May-2012 | 0.02 ± 0.01 | 2.18 ± 0.21$^{b}$ | 30.82 ± 3.58 |
| 4weekly*May-2012 | 0.02 ± 0.01 | 5.43± 0.26$^{a}$ | 39.98 ± 3.47 |
| ***Second growing season (Aug 2012 - May 2013)*** | | | |
| Grazing frequency | | | |
| 2 weekly | 0.03 ± 0.02 | 2.70 ± 1.26 | 20.23±9.67 |
| 4 weekly | 0.06 ± 0.04 | 3.35 ± 1.31 | 19.52±7.27 |
| Date | | | |
| Oct-2012 | 0.01 ± 0.01 | 0.44±0.09 $^{c}$ | 0.55±0.10 $^{c}$ |
| Jan-2013 | 0.09 ± 0.02 | 2.18 ± 0.53 $^{b}$ | 20.03±3.80 $^{b}$ |
| May-2013 | 0.04 ± 0.04 | 6.87 ± 0.41 $^{a}$ | 41.14±5.74 $^{a}$ |

$^{a, b,}$ superscripts indicate values within columns that are significantly different (P<0.05)

*In vitro* fermentation

The nutritive analyses of plantain and chicory substrates are presented in Table 3. Chicory and plantain from plots harvested on May 2013 had similar CP and HWSC. Differences in the content of ME, NDF, hemicellulose and OMD between plantain and chicory were found. NDF and hemicellulose concentrations were higher in plantain compared to chicory whereas ME, DOMD and HWSC:SC ratio were greater in chicory compared to plantain.

**Table 3**. Nutritive analysis of plantain and chicory from the 4 weeks grazing frequency plots, harvested in May 2013 and used as substrate (mean ± SEM; n=3)

| Nutritive value traits[1] | Plantain | Chicory |
|---|---|---|
| OM (g/kg DM) | 856.6 ± 4.5 | 850.9 ± 5.4 |
| Ash (g/kg DM) | 143.4 ± 4.5 | 149.1 ± 5.4 |
| CP (g/kg DM) | 298.9 ± 11.9 | 322.5 ± 6.4 |
| ME (MJ/kg DM) | 12.15 ± 0.1 $^{b}$ | 12.34 ± 0.4 $^{a}$ |
| NDF (g/kg DM) | 188.7 ± 5.6 $^{a}$ | 168.1 ± 5.2 $^{b}$ |
| ADF (g/kg DM) | 148.3 ± 4.4 | 136.5 ± 5.6 |
| Cellulose (g/kg DM) | 74.5 ± 3.1 | 67.5 ± 16.5 |
| Hemicellulose (g/kg DM) | 40.3 ± 1.4 $^{a}$ | 31.5 ± 0.9 $^{b}$ |
| Lignin (g/kg DM) | 73.8 ± 2.8 | 69.0 ± 11.1 |
| HWSC (g/kg DM) | 85.5 ± 3.2 | 83.4 ± 6.1 |
| OMD | 0.74 ± 0.5 $^{b}$ | 0.75 ± 2.7 $^{a}$ |

(continued)

| Nutritive value traits[1] | Plantain | Chicory |
|---|---|---|
| Ratio (HWSC:SC) | 0.75 ± $0.05^{b}$ | 0.91 ± $0.22^{a}$ |

[a, b] superscripts indicate values within rows that are significantly different (P<0.05)
[1] DM, dry matter; OM, organic matter; CP, crude protein; ME, metabolisable energy; NDF, neutral fibre detergent; ADF, acid fibre detergent; HWSC, hot water soluble carbohydrates; OMD, organic matter digestibility; HWSC:SC, the ratio of readily fermentable carbohydrates to structural carbohydrates.

Gas production parameters after 24 h of incubation

The *in vitro* gas production parameters after 24 h of incubation are reported in Table 4. Chicory (CH) and plantain (PL) had similar (P>0.05) potential gas production (A) but the total volume of gas produced after 24 hrs of incubation (V 24h) and the rate at which half the potential gas was produced ($R_{1/2A}$) were lower (P<0.05) for PL compared to CH.

The addition of acteoside increased (P<0.05) the potential gas production (A) of both PL and CH, and indeed the V 24h of both PL+36ac and CH+40ac was greater (P<0.05) than that of PL and CH alone. The addition of aucubin to CH (CH+10au; CH+20au) and PL (PL+10au) did not (P>0.05) impact the potential gas production (A) of either PL or CH or the total volume of gas produced after 24 hrs (V 24h). Although the addition of increasing concentrations of aucubin to the CH (CH+10au and CH+20au) caused a decrease (P<0.05) in the $R_{1/2A}$, the potential gas production and V 24h remains unchanged, aucubin may play a role in reducing the rate at which gas is produced in the rumen. This may perhaps partially explain the reduced A and V 24h observed for the PL *in vitro* fermentation compared to CH, however, the addition of extra aucubin to PL (PL+10au) did not result in reduction in gas production.

**Table 4**. *In vitro* gas production parameters of the treatments after 24 h of incubation (mean ± SEM; n=6).

| | Gas production parameters [1] | | | |
|---|---|---|---|---|
| *In vitro* Treatments[2] | A (mL/g DM) | $T_{1/2A}$ (h) | $R_{1/2A}$ (mL/h) | $V_{24h}$ (mL/g DM) |
| CH | 221.4 ± $3.21^{b}$ | 4.4 ± $0.17^{c}$ | 21.1 ± $0.85^{a}$ | 220.3 ± $3.29^{b}$ |
| CH+10au | 219.1 ± $3.53^{b}$ | 4.6 ± $0.09^{b}$ | 20.2 ± $0.46^{b}$ | 217.9 ± $3.47^{b}$ |
| CH+20au | 215.1 ± $2.37^{b}$ | 5.0 ± $0.09^{a}$ | 18.3 ± $0.51^{c}$ | 213.3 ± $2.39^{b}$ |
| CH+40ac | 237.5 ± $5.90^{a}$ | 4.5 ± $0.10^{c}$ | 21.5 ± $0.48^{a}$ | 235.4 ± $5.16^{a}$ |
| PL | 210.9 ± $1.40^{b}$ | 4.7 ± $0.22^{b}$ | 17.0 ± $0.67^{d}$ | 207.5 ± $1.42^{c}$ |
| PL+10au | 214.2 ± $2.15^{b}$ | 4.4 ± $0.16^{c}$ | 18.1 ± $0.59^{c}$ | 211.1 ± $1.77^{bc}$ |
| PL+36ac | 235.7 ± $7.90^{a}$ | 4.5 ± $0.12^{bc}$ | 18.8 ± $0.59^{c}$ | 229.9 ± $5.82^{ab}$ |

[1] Model parameters: A, potential gas production (mL/g DM); $T_{1/2A}$, time (h) at which half of A was reached; $R_{1/2A}$, rate (mL/h) at which $T_{1/2A}$ was reached; $V_{24h}$, total gas produced at 24h (mL/g DM).
[2] *In vitro* treatments: CH, chicory; CH+10au, chicory plus 10 mg aucubin /g DM; CH+20au, chicory plus 20 mg aucubin/g DM; CH+40ac, chicory plus 40 mg acteoside/g DM; PL, plantain (containing endogenous levels of 7 mg aucubin/g DM and 36 mg acteoside/g DM); PL+10au, plantain plus an additional 10 mg aucubin/g DM; PL+36ac, plantain plus an additional 36 mg acteoside/g DM.
[a, b] superscripts indicate values within columns that significantly differ (P<0.05).

pH, volatile fatty acid and ammonia concentration after 24 h of incubation

The *in vitro* incubation pH and end products after 24 h of incubation are reported in Table 5. The CH incubation had a higher pH and greater total VFA and $NH_3$ concentration (P<0.05; Table 5) compared to the PL incubation after 24 h. In regards to individual VFA production after 24 h, the CH incubation produced proportionally more (P<0.05) acetate and branched chain VFA (BCVFA: isobutyrate and isovalerate), and less (P<0.05) propionate and butyrate compared to the PL incubation (Table 5). This resulted in the CH incubation having a greater (P<0.05) acetate to propionate (A:P) ratio than the PL incubation.

The addition of aucubin and acteoside to either CH or PL did not (P>0.05) alter pH, total VFA or $NH_3$ concentration produced by CH and PL after 24 h; however, the proportion of individual VFA was affected in the *in vitro* treatments after 24 h. The addition of acteoside to both CH (CH+40ac) and PL (PL+36ac) decreased the proportion of acetate and increased the proportion of propionate produced after 24h, causing corresponding changes to A:P (P<0.05; Table 5).

The addition of aucubin did not (P>0.05) alter the proportion of acetate or propionate produced by CH or PL after 24 h of incubation. However, the proportion of acetate was lower when a level of 10 mg/g DM was added to chicory (CH+10au) compared to 20 mg/g DM (CH+20au).

The proportion of butyrate and BCVFA produced after 24 h of incubation was not affected (P<0.05) by adding aucubin or acteoside in both CH and PL. The addition of 10 mg aucubin /g DM (CH+10au) did not affect the proportion of BCVFA, but increasing the aucubin level at 20 mg/g DM (CH+20au) a reduction in the molar proportions of isobutyrate and isovalerate was observed in comparison to the pure CH incubation. Furthermore, the addition of acteoside to CH (CH+40ac) also resulted in a similar reduction of isobutyrate and isovalerate in comparison to the pure CH incubation.

**Table 5.** *In vitro* pH, total volatile fatty acids (VFA) concentration, molar proportion of individual VFA, ratio acetate to propionate (A:P), and ammonia ($NH_3$) concentration for chicory and plantain treatments after 24 h of incubation mean ± SEM; n=6).

| *In vitro* Treatments[1] | pH | Total VFA (mM) | Individual VFA (mmol/100 ml) | | | | | A:P | $NH_3$ mmol/g |
|---|---|---|---|---|---|---|---|---|---|
| | | | Acetate | Propionate | Butyrate | Isobutyrate | Isovalerate | | |
| | | | 68.15±0.22$^{ab}$ | | 8.28±0.22$^{b}$ | | | | 63.92±6.46$^{a}$ |
| CH | 6.48±0.01$^{a}$ | 65.94±1.09$^{a}$ | | 17.66±0.20$^{d}$ | 8.38±0.28$^{ab}$ | 1.78±0.03$^{a}$ | 2.86±0.02$^{a}$ | 3.86±0.04$^{a}$ | 58.28±5.95$^{a}$ |
| CH+10au | 6.48±0.01$^{a}$ | 64.54±1.94$^{a}$ | 67.75±0.30$^{bc}$ | 17.97±0.15$^{d}$ | 8.39±0.24$^{ab}$ | 1.80±0.04$^{a}$ | 2.82±0.03$^{a}$ | 3.77±0.02$^{b}$ | 56.43±3.56$^{a}$ |
| CH+20au | 6.48±0.01$^{a}$ | 64.97±1.19$^{a}$ | 68.50±0.21$^{a}$ | 17.52±0.15$^{d}$ | | 1.71±0.01$^{b}$ | 2.66±0.06$^{b}$ | 3.91±0.04$^{a}$ | |
| CH+40ac | 6.47±0.01$^{a}$ | 66.35±0.86$^{a}$ | 67.35±0.20$^{c}$ | 18.57±0.14$^{c}$ | 8.12±0.21$^{b}$ | 1.68±0.02$^{b}$ | 2.65±0.03$^{b}$ | 3.63±0.03$^{c}$ | 64.43±4.03$^{a}$ |
| | | | | | | | | | 42.64±7.97$^{b}$ |
| PL | 6.43±0.01$^{b}$ | 61.18±2.22$^{b}$ | 67.28±0.28$^{c}$ | 19.01±0.15$^{b}$ | 8.77±0.04$^{a}$ | 1.34±0.02$^{c}$ | 2.18±0.05$^{c}$ | 3.54±0.03$^{d}$ | 34.14±2.21$^{b}$ |
| PL+10au | 6.42±0.01$^{b}$ | 64.17±1.04$^{ab}$ | 67.31±0.19$^{c}$ | 19.25±0.15$^{b}$ | 8.66±0.06$^{a}$ | 1.30±0.03$^{c}$ | 2.11±0.07$^{c}$ | 3.50±0.03$^{d}$ | |
| PL+36ac | 6.41±0.01$^{b}$ | 64.30±0.64$^{ab}$ | 66.39±0.31$^{d}$ | 19.92±0.10$^{a}$ | 8.53±0.07$^{a}$ | 1.28±0.02$^{c}$ | 2.07±0.06$^{c}$ | 3.33±0.03$^{e}$ | 37.72±3.40$^{b}$ |

$^{a, b, c, d}$ Superscripts letters indicate values within columns that are significantly different ($P<0.05$). SEM, standard error of the mean. [1] *In vitro* treatments: CH, chicory; CH+10au, chicory plus 10 mg aucubin /g DM; CH+20au, chicory plus 20 mg aucubin/g DM; CH+40ac, chicory plus 40 mg acteoside/g DM; PL, plantain (containing endogenous levels of 7 mg aucubin/g DM and 36 mg acteoside/g DM); PL+10au, plantain plus an additional 10 mg aucubin/g DM; PL+36ac, plantain plus an additional 36 mg acteoside/g DM.

***Volatile fatty acid production over time***

VFA production over time (mmol/g DM) of acetate (Figure 1A), propionate (Figure 1B), total VFA (Figure 1C), and the BCVFA (isobutyrate and isovalerate) (Figure 2B) showed to be different between the *in vitro* treatments ($P < 0.001$); however, butyrate (Figure 1C) production was similar ($P > 0.05$) between the *in vitro* treatment.

Over 24 hours of incubation the CH incubation produced more acetate, BCVFA, and total VFA, but similar propionate and butyrate production compared to the PL incubation. The addition of aucubin and acteoside to CH and PL affected acetate, propionate, and total VFA production (Figure 1); while butyrate and BCVFA production (Figure 2) were not modified by adding aucubin or acteoside to CH or PL.

Acetate production (Figure 1A) in CH and PL was not affected by adding aucubin or acteoside to either CH (CH+10au; CH+20au; CH+40ac) or PL (PL+10au; PL+36ac) substrates. Whereas, the addition of acteoside to both chicory (CH+40ac) and plantain (PL+36ac) increased propionate production (Figure 1B) compared to all *in vitro* treatments. Nonetheless, the addition of acteoside to chicory (CH+40ac) increased the total VFA production (Figure 1C) by CH, but not in PL (PL+36ac).

The aucubin level of 20 mg/g DM added in chicory (CH+20au) produced less acetate, propionate and a lower total VFA compared to chicory with acteoside (CH+40ac) incubation. Moreover, the CH+20au treatment had an acetate and total VFA production similar to plantain (PL; PL+10au; PL+36ac) treatments and a propionate production similar to PL+10au and PL+36ac treatments.

**Ammonia production over time**

The net $NH_3$ production (mmol/g DM) increased ($P<0.001$) over time in plantain and chicory *in vitro* treatments (Figure 3). Over 24 hours of incubation plantain with the natural concentration of bioactive compounds (7 and 36 mg/g DM of aucubin and acteoside, respectively) produced 40% less $NH_3$ compared to chicory. During the first 5 hours of incubation there were no differences in the net $NH_3$ production between plantain and chicory substrates, but after 8 hours plantain started to produce less $NH_3$ compared to chicory. However,

Surprisingly the addition of aucubin (particularly at 10 or 20 mg/g DM) and acteoside (particularly at 40 mg/g DM) into chicory have the impact of decreasing the net $NH_3$ production when compared to the pure chicory incubation. We found that the greater level of aucubin (20 mg/g DM) in chicory tends ($P=0.053$) to produce less $NH_3$ when compared to 10 mg aucubin/g DM (CH+10au) in chicory. In plantain substrate, the addition of aucubin (PL+10au) remained a similar $NH_3$ production whereas the addition of acteoside in plantain (PL+36ac) decreased the net $NH_3$ production when compared to plantain.

The addition of 20 mg aucubin/g DM in chicory (CH+20au) had the stronger effect reducing $NH_3$ production when compare to chicory treatments (CH; CH+10au; CH+40ac) with a $NH_3$ production similar to plantain (PL) and plantain with extra aucubin (PL+10au). While, plantain with extra acteoside (PL+36ac) was the treatment that most reduced the net $NH_3$ production over 24 hours.

Discussion:

Bioactive compounds in plantain

The increase of aucubin and acteoside concentration in plantain swards throughout the growing season has been reported. The concentration of these compounds in plantain has been reported to be influenced by genetic and environmental factors, as well as leaf age. The very low levels of catalpol detected in plantain showed that the cultivar Ceres Tonic is near absent of this iridoid glycoside. Catalpol is biologically synthesised from its precursor aucubin. Hence, during the selection of the cultivar Ceres Tonic the pathway from aucubin to catalpol has been reduced to a very low basal rate of synthesis, although catalpol has been found in natural ecotypes of plantain and in the cultivar Grassland Lancelot.

The concentrations of aucubin and acteoside increased similarly in both growing seasons and the highest concentration of both compounds was in autumn (May 2012 and May 2013). The concentrations of aucubin found in this study were lower to those reported earlier. High concentrations of up to 30 mg/g of aucubin depending on the genotype, and from 10 to 27 mg/g DM for the cultivar Ceres Tonic have been reported. Whereas, acteoside can be present at 60 to 90 mg/g DM in natural ecotypes and at 15 to 41mg/g DM in the cultivar Ceres Tonic, which are similar concentrations to those found in the present study. In contrast, concentrations as low as 3.2 mg/g DM of acteoside in the cultivar Ceres Tonic have been found previously.

Ruminal fermentation

In our study plantain containing natural levels of aucubin and acteoside (7 and 36 mg/g DM, respectively) had

the same potential for gas production (GP) as chicory. Differences in fermentation pattern are attributable to differences in the nutritive characteristics between substrates in the type of carbohydrate and its rate of depolymerisation. The higher ME, OMD, ratio RSC:SC and the lower NDF in chicory compared to plantain suggests chicory disintegrates more rapidly in the rumen than plantain. Fast ruminal degradation has been reported for both herbs with DM loss of 0.26 and 0.25 %/hour for chicory and plantain, respectively. Although, our results suggest that plantain had slower rumen degradability compared to chicory due to its rate ($R_{1/2A}$) of GP which is directly proportional to the rate of degradation, and because after 24 hour of incubation the volume of gas (V 24h) produced was lower in plantain than chicory. Consequently, the total VFA concentration and production was greater in chicory than in plantain. Plantain produced a lower total VFA and acetate than chicory after 24 hours of incubation. Similarly, *in vivo* experiments with deer have shown a trend for total VFA and acetate to be less when deer were fed plantain compared to ryegrass. Although, the higher NDF and hemicellulose content in plantain compared to chicory might encourage the growth of acetate producing bacteria, high pectin levels have been reported for chicory, and pectin fermentation produces more acetate.

VFA are the end products of rumen fermentation and represent the main supply of ME for ruminants, and a reduction in their production is considered nutritionally unfavorable for the animal. The feeding value (FV) for chicory has been always consistently superior for ruminants, while the FV for plantain has shown variable results throughout the growing season. This seasonal variation for plantain has been attributed to changes in the voluntary feed intake (VFI) and OMD, with the higher FV reported for plantain during summer and autumn.

Acteoside increased GP and propionate production whereas aucubin did not modify any fermentation parameter when compared with the controls. The increase in GP with acteoside reflects an increase in fermentable substrate. Rumen microbes might be either capable of degrading or able to tolerate its effect due to containing non-specific glycosidase that might break the compound down. Aucubin is an iridoid glycoside with an O-linked glucose at C-1 and the sugar can be hydrolysed by *B*-glucosidase forming the aglycone of aucubin - aucubigin. While, acteoside is a phenylpropanoid (with caffeic acid) and phenylethanoid (with hydroxytyrosol) glycoside. Acteoside always has glucose as the central sugar, but rhamnose the other carbohydrate may be replaced, or be present as additional auxiliaries.

It has been reported that both gram-positive (most acetate- and butyrate-producing bacteria) and gram-negative bacteria (normally propionate-producing bacteria) are inhibited by carvacrol (phenolic terpenoid) in a dose-dependent manner. However, our results suggest that the sugar components from acteoside were used as an energy source to produce propionate either in chicory and plantain. Propionate is produced via the succinate: propionate pathway by some ruminal bacterial and carbon dioxide ($CO_2$) is formed increasing GP. Conversely, aucubin aglycone has been identified as affecting bacteria and fungi, by binding to free amino acid making them unavailable and contributing to its biological and toxic effect. Therefore, the opposing effect between CH+20au and CH+40ac suggests that aucubin resulted in a general inhibition of rumen microbial fermentation due to the negative effect on acetate and total VFA production. It may be possible that aucubin negatively affected acetate-producing bacteria thereby exhibiting a bactericidal effect.

Ammonia production

Both compounds (aucubin and acteoside) reduced the net $NH_3$ production in chicory and plantain. The net $NH_3$ production indicates the magnitude at which the protein degradation exceeds the capacity for microbial utilisation and the likelihood that plant N is insufficient for microbial growth. Since the majority of rumen bacteria use ammonia as their N source, the availability of ammonia is important for microbial protein production, and the microbial yield of VFA may be restricted by ammonia insufficiency. However, ammonia concentration was always above the minimum requirement (5 mg/dl) for microbial growth in our incubations.

Chicory has been described as a forage crop that reduces $NH_3$ concentration in the rumen when compared to cocksfoot (*Dactylis glomerata* L.) and perennial ryegrass. The higher ratio of RFC:SC in chicory would provide increased energy for microbial growth. The ammonia N disappearance with increased energy availability has been assumed to be due to its incorporation into microbial protein. Microbial $NH_3$ utilisation in the rumen is primarily carbohydrate-limited, and increasing carbohydrate availability in the rumen has the potential of improving the efficiency of microbial utilisation of $NH_3$. However, we observed that plantain produced less $NH_3$ than chicory after 24 h of incubation, consistent with the net ammonia ($NH_3$/g DM) production over time.

It has been suggested that a reduction in $NH_3$ by deamination is associated with a reduction of the BCVFA (isobutyrate and isovalerate), acids which are derived from amino acid catabolism in the rumen. The observed reduction in the molar proportion of BCVFA in chicory with aucubin (20 mg/g) and acteoside (40mg/g) suggests that both compounds reduced amino acid deamination. Hence, an inhibition of amino acid deamination has practical implications because it may increase ruminal escape of dietary protein and improve the efficiency of N use. However, as the BCVFA production over time was not affected by the addition of aucubin and acteoside to any substrate, the changes in the proportion molar of the BCVFA would be a consequence of the greater total VFA production in chicory with acteoside (CH+40ac) and lower total VFA production in chicory with 20 mg/g of aucubin (CH+20au).

The reduced $NH_3$ concentration in this study was unlikely to be a consequence of an increased utilisation for

microbial synthesis, due to the proposed antimicrobial properties of these compounds. Aucubin aglycone has been identified as affecting bacteria and fungi. That aucubin added to chicory (20 mg/g) had the same effect as plantain in decreasing $NH_3$ production may be a consequence of the bactericide effect of this compound. In contrast, although acteoside has also been described as antimicrobial and antifungal, its mechanism of action is not clear. As such, the antimicrobial action by acteoside in our study appears less likely than a reduction of $NH_3$ due to greater fermentation resulting from the ingestion of acteoside.

Conclusions

The bioactive compounds present in plantain were shown to reduce the net $NH_3$ production. Acteoside improved rumen fermentation by increasing propionate production and total VFA, while aucubin did not modify any VFA parameter. The antagonist effect between aucubin and acteoside evaluated in chicory showed that aucubin is mainly a bactericide and acteoside an energy source. Both compounds (aucubin and acteoside) reduced the net $NH_3$ production in chicory and plantain. Decreasing net $NH_3$ production may lead to a reduction in the proportion of total nitrogen ingested by an animal that is excreted in the urine of the animal.

**Animal trials**

**Experiment 1**

*Experimental*

The experiment was carried out on kale forages at PGG Wrightson Seeds' Marshdale trial site near Oxford, New Zealand over 2 years. In year 1, ninety Friesian x Jersey replacement dairy heifers (approx.180 kg liveweight) from a neighbouring dairy herd were used for this experiment. The herd was divided into 3 reps of 15 calves for each of the two treatments.

In year 2, 80 heifers were randomly allocated to two groups and stocked onto one of the two treatments.

Heifers were grazed on kale (cv Sovereign) for 56 days between 21 June and 16 August 2013 in year 1 or 20 June and 15 August 2014 in year 2. The treatments were 2.5 kg DM kale/100kg Lwt/day and 3 kg DM/cow/day of either grass balage or plantain balage.

The grass balage did not contain detectable quantities of secondary plant metabolites suitable for use in the invention. The plantain balage contained aucubin and acteoside as outlined below in Table 7.

Balages were selected to be as close to iso-nitrogenous as possible. The heifers were allocated their silage and kale together at 9 am and were offered a fresh break of kale every day with their allocated area determined by measurements of crop yield.

Throughout the 8 weeks of the trial measurements were made of: pre and post grazing masses of kale (from which utilisation and apparent intake were calculated), bale weight and liveweight change of heifers. Heifers were adapted to crops over a 7-day period (week 0).

Spot samples of urine and faeces were collected on one day in weeks 3 and 8 of the trial in year 1 and in weeks 2, 4, 6 and 8 in year 2. Samples were collected from all 90 (year 1) or 80 heifers (year 2) between 11 am and 1 pm. Urine samples were analysed for total N, creatinine, urea-N and ammonia N. Faecal samples were analysed for total N. The liveweight measurement and urine and faecal samples were collected in a set of cattle yards located next the kale paddocks.

Samples of kale, silage and balage were analysed for forage composition using NIRS and secondary plant compound concentration determined by HPLC.

Treatment effects on N intake and excretion for sampling days in Year 1 and 2 are given in Table 6.

Table 6. *Nitrogen (N) intake and excretion in urine and creatinine concentration of dairy heifers grazing kale in 2 years.*

| Treatment (Year 1) | N-Intake (g/cow/day) | $NH_3$ (nmol/l) | Urea (nmol/l) | Urinary – N (%) | Creatinine (mmol/l) |
|---|---|---|---|---|---|
| 12 July | | | | | |
| Kale + grass balage | 191 | | | 0.41 | 2.27 |
| Kale + plantain balage | 197 | | | 0.48 | 2.72 |
| | | | | | |
| 16 Aug | | | | | |
| Kale + grass balage | 202 | | | 0.44 | 0.28 |
| Kale + plantain balage | 226 | | | 0.24 | 0.12 |
| Treatment (Year 2) | N-Intake (g/cow/day) | $NH_3$ (nmol/l) | Urea (nmol/l) | Urinary – N (%) | Creatinine (mmol/l) |
| 4 July | | | | | |
| Kale + grass balage | 158 | 3.5 | 94 | 0.45 | 3.3 |
| Kale + plantain balage | 158 | 1.9 | 61 | 0.31 | 3.1 |
| | | | | | |
| 18 July | | | | | |
| Kale + grass balage | 155 | 0.53 | 116 | 0.59 | 4.1 |
| Kale + plantain balage | 165 | 0.48 | 43 | 0.25 | 2.1 |
| | | | | | |
| 1 Aug | | | | | |
| Kale + grass balage | 169 | 1.84 | 171 | 0.76 | 5.6 |
| Kale + plantain balage | 194 | 1.31 | 109 | 0.51 | 4.4 |
| | | | | | |
| 15 Aug | | | | | |
| Kale + grass balage | 207 | 1.02 | 106 | 0.52 | 3.7 |
| Kale + plantain balage | 199 | 1.49 | 69 | 0.37 | 4.2 |

In year 1, the first sampling period showed no difference in urinary N concentration but in the second sampling period urinary N concentration was reduced from 0.44 to 0.24%, a reduction of 45% despite having numerically high N intake. Lower urinary N concentration corresponded to a reduction in creatinine of a similar magnitude.

In year 2, urinary N concentration was lower at all sampling periods by between 35 and 62% with urinary N concentration decreasing from 0.58 to 0.36% on average. There was a corresponding decrease in creatinine concentration at some but not all samples.

Secondary plant compound concentrations differed between lines of plantain balage used in Year 2 - see Table 7. Line M, which was fed during the first two sampling periods had a higher acteoside concentration than other lines and line S, fed for the rest of the trial had elevated aucubin concentrations. Line X was not used.

Table 7. *Concentration of aucubin, catalpol and acteoside in lines of Tonic plantain balage used in Year 2.*

| | Catalpol | Aucubin | Acteoside |
|---|---|---|---|
| Line | (mg/g) | (mg/g) | (mg/g) |
| M | ND | 0.01 | 2.40 |
| Kim S | ND | 0.17 | 0.95 |
| Kim X | ND | 0.07 | 1.00 |

*Discussion*

In Year 1, offering plantain balage reduced urinary-N (%) concentration in the second but not the first measurement. Creatinine concentration suggested reduction in urinary-N concentration was largely due to changes in urine volume. Variable urinary-N concentrations between sampling times may be a consequence of variation in secondary

plant compound concentration in individual bales. However, these were not measured in Year 1.

In Year 2, urinary-N was decreased relative to the ryegrass control at all time periods in herds offered plantain balage. Reduction in urinary N concentration was not always associated with a reduction in creatinine concentration suggesting changes in urine volume were not the only mechanisms leading to this decrease.

During the first two sampling periods the M line of plantain balage was used while the Kim S line was used for the remainder of the study. The M line had a higher concentration of acteoside than the other lines and this suggests it may play a role in reducing urinary N concentration.

This field study provides evidence that plantain (in balage form) at about 25% of the diet reduces urinary N concentration by 35% despite a similar N intake.

**Experiment 2**

**Indoor dairy heifer study**

**Objective:**

Investigate effect of feeding either plantain or ryegrass pasture on N utilisation modulation when DMI is fixed.

**Materials and methods**

*Design and management*

The study was undertaken at Lincoln University Research Dairy Farm, Canterbury, New Zealand under the authority of the Lincoln University Animal Ethics Committee (#296-13). Twelve 8-9 month Holstein-Friesian dairy heifers were used. Heifers grazed on perennial ryegrass/white-clover pasture prior to the study were equally divided into two dietary treatment groups (ryegrass pasture or plantain) based on their liveweight and breeding worth (BW). The study consisted of a 13 days adaptation period and a 3 day N study period. Heifers were given the same daily grazing allowance (on a DM basis), calculated according to the requirement for maintenance × 1.5 during adaptation period. Four days prior to measurement period, heifers were adapted to the stalls where they were housed individually with pre-designed urine harness attached.

Freshly cut forage was offered to individual heifer in metabolism stalls at 1000 h and 1300 h, daily. All heifers received the same feed allowance (on a DM basis) during N balance study period, calculated according to the requirement for maintenance × 1.5. Fresh water was available at all times throughout the study.

*Feed and water measurement and analysis*

Intake was measured daily per heifer by weighing offered and refused forage. Forage samples (500 g fresh weight) were collected at feeding and oven-dried at 65 °C to determine DM%, before grinding through a 1.0-mm sieve (Christy Lab Mill; Suffolk; UK). Forage samples were analysed by Near Infra-Red Spectroscopy (NIRS systems 6500; feed TECH; New Zealand) for predicted crude protein (CP), organic matter digestibility, acid detergent fibre, neutral detergent fibre, and soluble sugar and starch content. Metabolisable Energy (ME) content was derived from predicted organic matter digestibility on the basis of an *in vitro* cellulase digestibility assay, calibrated against *in vivo* standards.

Daily water intake was measured for each individual heifer daily by recording water volumes removed from the drinking bucket.

*Plasma sampling and analysis*

Two 10 ml blood sample were collected using a Li-heparinised evacuated tube from the jugular vein of each cow at 1400 h daily while in the met stall. Plasma was harvested following centrifugation at 1200 × g for 12 min at 4 °C. At the end of the collection period, plasma samples were pooled per heifer and stored at -20 °C. Plasma urea N (PUN) was quantified using a Daytona RX clinical analyser (Randox; Nishinomiya; Japan).

*Faeces and urine collection and analysis*

Total output of urine from each heifer was collected three times a day at 0930, 1430, and 1930 h using the externally applied urine harness attached with a pipe connector. Urine drained into individual container containing hydrochloric acid placed under the crate to ensure urine pH < 3 in order to prevent N volatilisation.

Three times a day at 0930, 1430, and 1930 h spot urine sample were collected and then stored at -20 °C. Daily

faeces was collected from behind the animal at various intervals through the day. Faecal sample was oven-dried at 65 °C to determine DM%. Representative liquid urine and oven-dried faeces from five days bulked samples per heifer was sub sampled and measured for N concentration using a Variomax CN Analyser (Elementar Analysensysteme GmbH; Hanau; Germany).

**Results**

*N balance*

Despite similar DM intake and nitrogen intake, heifers fed plantain had significantly lower urinary-N concentration (%), lower total N excreted (g/day) and therefore a greater N utilisation efficiency, as shown in Table 8. Further, heifers on plantain had a lower plasma urea concentration consistent with a difference in N utilisation (partitioning).

Table 8. *Nitrogen partitioning in heifers.*

| | **Pasture** | **Plantain** |
|---|---|---|
| DMI (kg/day) | 4.90 | 5.23 |
| N intake (g/day) | 119.9 | 125 |
| Urine N% | 0.35 | 0.14 |
| Urine N (g/day) | 65.4 | 40.5 |
| Intake N in urine (%) | 54% | 32% |
| Plasma urea (g/L) | 0.25 | 0.15 |

**Experiment 3**

**Indoor sheep study Ia**

**Sheep**

Animals used in this study were 6 month old Romney × Southdown ewe hoggets. Prior to the study they were grazed on predominantly ryegrass/white clover pasture near Lincoln, Canterbury, New Zealand (Kimihia Research Centre). Two weeks prior to the experiment they were moved to their experimental diets grazed *in situ* and subsequently transported to Johnstone Memorial Laboratory, Lincoln University where they were held indoors for 7 days in individual metabolism crates in one of two rooms.

The sheep were allocated to one of three treatment groups (n = 7 sheep) that were balanced for live weight (average 38 kg). Treatments were either plantain (*Plantago lanceolata, cv* Ceres Tonic or breeding line WDA+) or perennial ryegrass (*Lolium perenne*, cultivars Base and Expo) offered at a rate so that each sheep had feed left prior to the following morning's feed. Refused feed from the previous day was collected prior to feeding and weighed, and a sub sample taken for DM analysis. Sheep at access to drinking water from buckets which had known volumes of water from which water consumed was calculated. All procedures involving these animals were approved by the Lincoln University Animal Ethics Committee.

**Urine and faeces**

Urine and faecal matter were collected separately into collection trays and buckets situated under the grating of each metabolism crate. To prevent cross-contamination net separators (shade cloth stretched over a metal frame and fixed underneath the metabolism crates) were used to trap faeces and allow urine to pass through the mesh.

Urine was acidified to prevent nitrogen losses by adding 250 mL of 5% $H_2SO_4$ solution to the collection trays. Daily urine volume for each sheep was measured in a volumetric flask and a 70 mL sample was placed into a polypropylene container and stored in a freezer. The collection trays were rinsed with tap water, returned to each metabolism crate and left until a non-acidified (fresh) sample of (70 mL) urine could be collected. Acid was then placed in the tray for urine collected overnight. Fresh and acidified urine samples were bagged separately in plastic bags and stored frozen for subsequent analyses.

The metabolism crates were cleaned daily to collect all faecal matter and each sheep's daily faecal production was weighed. A small sample was set aside to be freeze dried for subsequent analysis. The rest was divided between

two perforated plastic bags which were weighed and placed in 90 °C ovens for 48 h to dry. Drying time depended on the size of the sample, therefore if a sample did not dry in the allocated time it was left for a further 48 h. Once dry, the bags were re-weighed and the dry matter percentage calculated (i.e. dry weight/wet weight × 100). This gave two values for faecal dry matter percentage (DM %) for each sheep's daily output. The mean of these two DM% values was applied to the total faecal weight to determine faecal water content.

**Results**

Despite similar DM and nitrogen intake, lambs on *cv.* Ceres Tonic had a significantly lower urinary-N concentration and a smaller percentage (15 vs 32%) of dietary N was excreted in the urine. The WDA+ lambs partitioned N in a way more similar to ryegrass.

Table 9. Nitrogen intake and urinary N excretion of lambs grazing 3 different forages.

| **Treatment** | **DM Intake (g DM/day)** | **N intake (g)** | **Urinary N (%)** | **% diet N in urine (%)** |
|---|---|---|---|---|
| Grass | 1069 | 29 | 0.32 | 41 |
| Tonic | 935 | 27 | 0.15 | 32 |
| WDA+ | 898 | 29 | 0.29 | 36 |

**Discussion**

*N partitioning*

In spite of having similar intakes of nitrogen, lambs grazing *cv.* Ceres Tonic had less N in urine and therefore a greater N utilisation efficiency. A lower proportion of dietary N in urine is consistent with the lower ammonium levels of *cv.* Ceres Tonic plantain in the *in vitro* incubations reported herein and the lower blood plasma urea levels reported for dairy cattle on *cv.* Ceres Tonic plantain diets (Heifer indoor study reported herein). A similar difference in N utilisation efficiency was noted for cattle grazing *cv.* Ceres Tonic (see heifer study herein).

**Claims**

1. Use of a secondary plant metabolite to reduce the amount of nitrogen excreted in the urine of an animal, wherein the secondary plant metabolite is selected from acteoside and catalpol.

2. Use of a secondary plant metabolite to reduce the amount of nitrate derived from an animal that is leached into groundwater and/or surface water, wherein the secondary plant metabolite is selected from acteoside and catalpol.

3. Use of a secondary plant metabolite to reduce nitrogenous gases derived from an animal, wherein the secondary plant metabolite is selected from acteoside and catalpol.

4. Use of a secondary plant metabolite to reduce urinary nitrogen concentration in an animal, wherein the secondary plant metabolite is selected from acteoside and catalpol.

5. Use of a secondary plant metabolite to reduce nitrogen volatilisation from a pastoral system, wherein the secondary plant metabolite is selected from acteoside and catalpol.

6. Use of a secondary plant metabolite to reduce greenhouse gas emissions from a pastoral system, wherein the secondary plant metabolite is selected from acteoside and catalpol.

7. Use of a secondary plant metabolite to reduce nitrate leaching from a pastoral system, wherein the secondary plant metabolite is selected from acteoside and catalpol.

8. Use according to any one of claims 1 to 7 wherein the secondary plant metabolite is acteoside.

9. Use according to any one of claims 1 to 8 wherein the secondary plant metabolite is derived from a plant of the genus *Plantago.*

10. Use according to any one of claims 1 to 9 wherein the secondary plant metabolite is derived from a plant of the species *Plantago lanceolata.*

## Patentansprüche

1. Verwendung eines sekundären Pflanzenmetaboliten zur Verringerung der im Urin eines Tieres ausgeschiedenen Stickstoffmenge, wobei der sekundäre Pflanzenmetabolit aus Acteosid und Catalpol ausgewählt ist.

2. Verwendung eines sekundären Pflanzenmetaboliten zur Verringerung der Nitratmenge tierischen Ursprungs, die in das Grundwasser und/oder Oberflächenwasser ausgewaschen wird, wobei der sekundäre Pflanzenmetabolit aus Acteosid und Catalpol ausgewählt ist.

3. Verwendung eines sekundären Pflanzenmetaboliten zur Verringerung von stickstoffhaltigen Gasen tierischen Ursprungs, wobei der sekundäre Pflanzenmetabolit aus Acteosid und Catalpol ausgewählt ist.

4. Verwendung eines sekundären Pflanzenmetaboliten zur Verringerung der Urinstickstoffkonzentration bei einem Tier, wobei der sekundäre Pflanzenmetabolit aus Acteosid und Catalpol ausgewählt ist.

5. Verwendung eines sekundären Pflanzenmetaboliten zur Verringerung der Stickstoffverflüchtigung aus einem Weidesystem, wobei der sekundäre Pflanzenmetabolit aus Acteosid und Catalpol ausgewählt ist.

6. Verwendung eines sekundären Pflanzenmetaboliten zur Verringerung der Treibhausgasemissionen aus einem Weidesystem, wobei der sekundäre Pflanzenmetabolit aus Acteosid und Catalpol ausgewählt ist.

7. Verwendung eines sekundären Pflanzenmetaboliten zur Verringerung der Nitratauswaschung aus einem Weidesystem, wobei der sekundäre Pflanzenmetabolit aus Acteosid und Catalpol ausgewählt ist.

8. Verwendung nach einem der Ansprüche 1 bis 7, wobei der sekundäre Pflanzenmetabolit Acteosid ist.

9. Verwendung nach einem der Ansprüche 1 bis 8, wobei der sekundäre Pflanzenmetabolit aus einer Pflanze der Gattung Plantago gewonnen wird.

10. Verwendung nach einem der Ansprüche 1 bis 9, wobei der sekundäre Pflanzenmetabolit von einer Pflanze der Spezies Plantago lanceolata abgeleitet ist.

## Revendications

1. Utilisation d’un métabolite végétal secondaire pour réduire la quantité d’azote excrété dans l’urine d’un animal, dans laquelle le métabolite végétal secondaire est choisi parmi de l’actéoside et du catalpol.

2. Utilisation d’un métabolite végétal secondaire pour réduire la quantité de nitrate dérivé d’un animal qui s’infiltre dans les eaux souterraines et/ou les eaux de surface, dans laquelle le métabolite végétal secondaire est choisi parmi de l’actéoside et du catalpol.

3. Utilisation d’un métabolite végétal secondaire pour réduire des gaz azotés dérivés d’un animal, dans laquelle le métabolite végétal secondaire est choisi parmi de l’actéoside et du catalpol.

4. Utilisation d’un métabolite végétal secondaire pour réduire une concentration d’azote urinaire chez un animal, dans

laquelle le métabolite végétal secondaire est choisi parmi de l'actéoside et du catalpol.

5. Utilisation d'un métabolite végétal secondaire pour réduire une volatilisation d'azote à partir d'un système pastoral, dans laquelle le métabolite végétal secondaire est choisi parmi de l'actéoside et du catalpol.

6. Utilisation d'un métabolite végétal secondaire pour réduire des émissions de gaz à effet de serre à partir d'un système pastoral, dans laquelle le métabolite végétal secondaire est choisi parmi de l'actéoside et du catalpol.

7. Utilisation d'un métabolite végétal secondaire pour réduire une infiltration de nitrate à partir d'un système pastoral, dans laquelle le métabolite végétal secondaire est choisi parmi de l'actéoside et du catalpol.

8. Utilisation selon l'une quelconque des revendications 1 à 7 dans laquelle le métabolite végétal secondaire est de l'actéoside.

9. Utilisation selon l'une quelconque des revendications 1 à 8 dans laquelle le métabolite végétal secondaire est dérivé d'un végétal du genre *Plantago.*

10. Utilisation selon l'une quelconque des revendications 1 à 9 dans laquelle le métabolite végétal secondaire est dérivé d'un végétal de l'espèce *Plantago lanceolata.*

A)

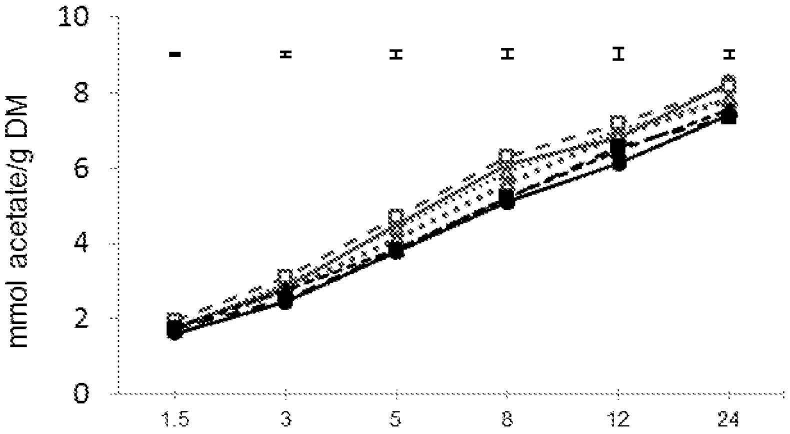
10
8
6
4
2
0
mmol acetate/g DM
1.5
3
5
8
12
24

B)

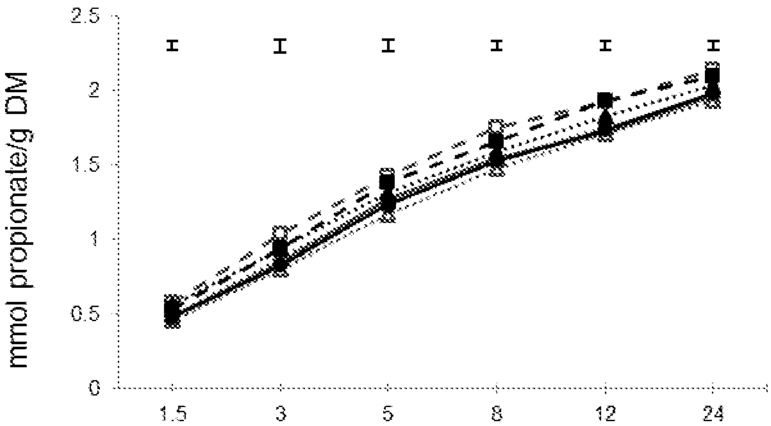
2.5
2
1.5
1
0.5
0
mmol propionate/g DM
1.5
3
5
8
12
24

C)

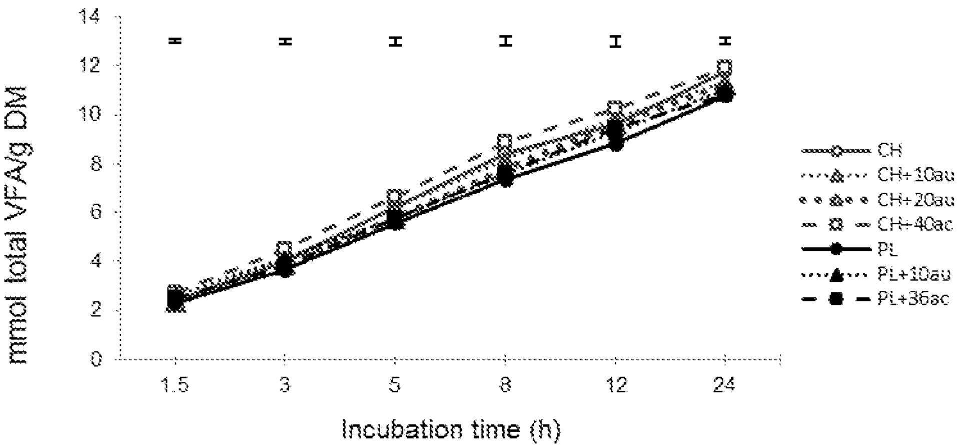
14
12
10
8
6
4
2
0
mmol total VFA/g DM
1.5
3
5
8
12
24
Incubation time (h)
CH
CH+10au
CH+20au
CH+40ac
PL
PL+10au
PL+36ac

Figure 1

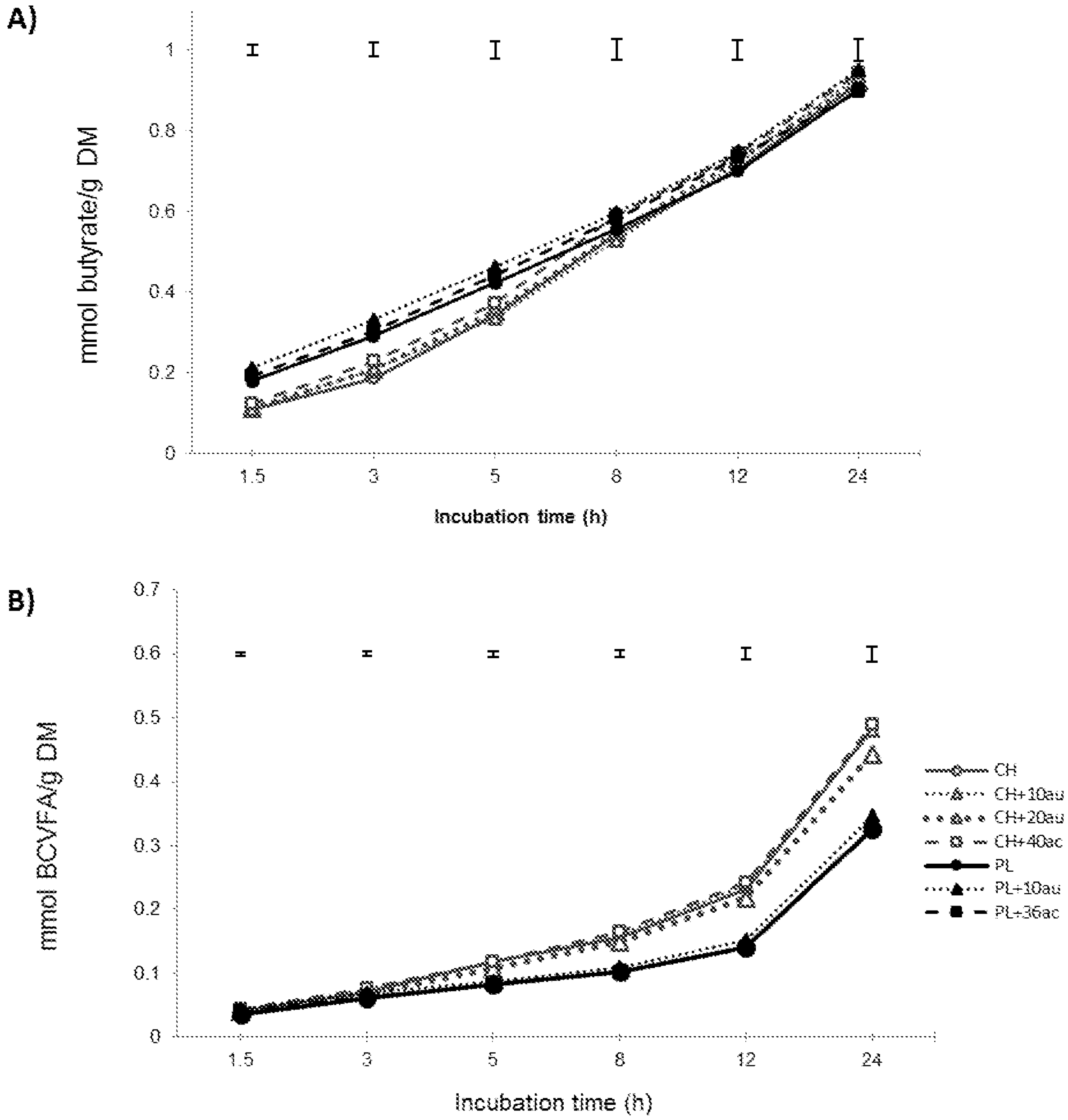
A)
mmol butyrate/g DM
0
0.2
0.4
0.6
0.8
1
1.5
3
5
8
12
24
Incubation time (h)
B)
mmol BCVFA/g DM
0
0.1
0.2
0.3
0.4
0.5
0.6
0.7
1.5
3
5
8
12
24
Incubation time (h)
CH
CH+10au
CH+20au
CH+40ac
PL
PL+10au
PL+36ac

Figure 2

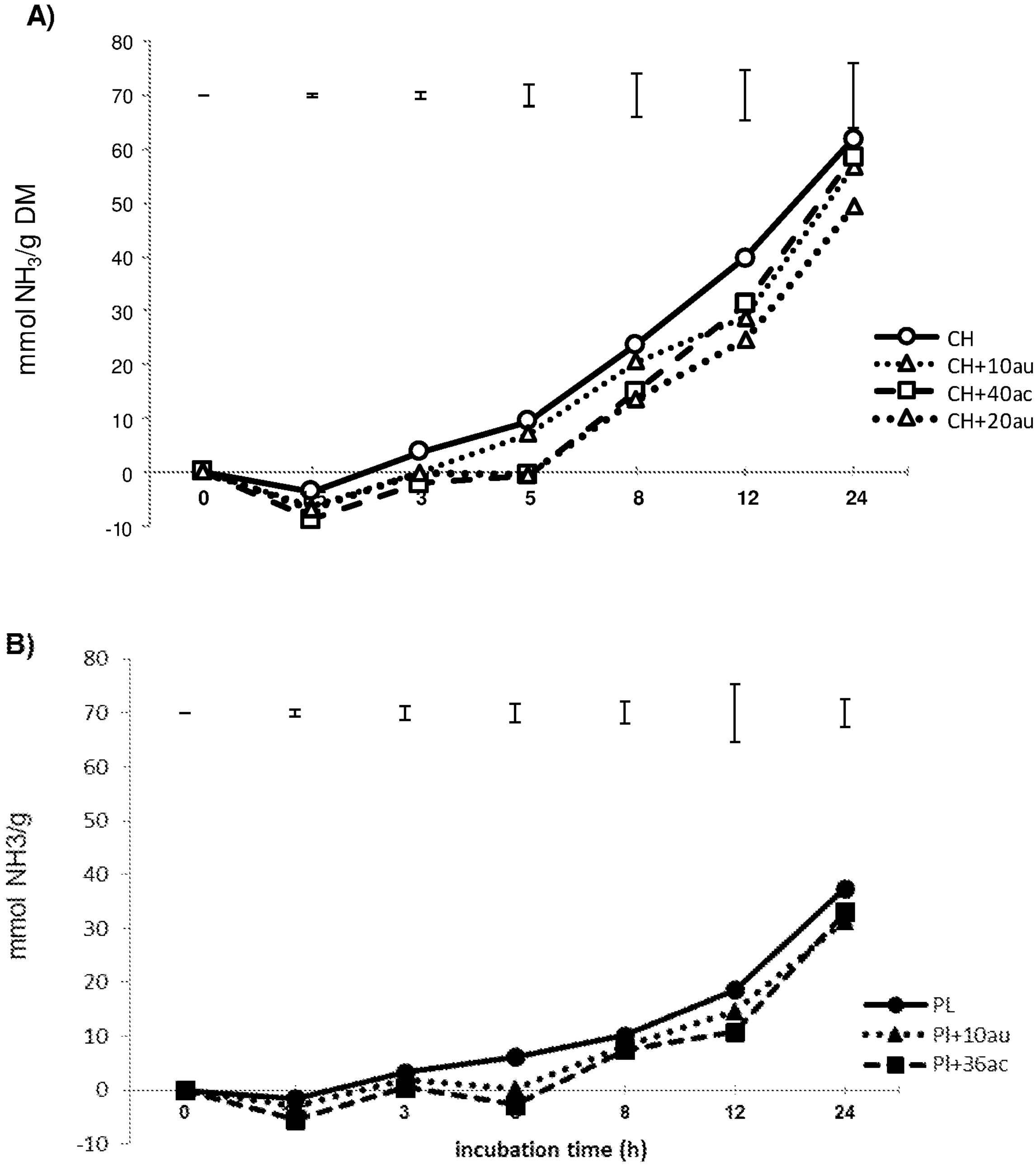
A)
80
70
60
50
40
30
20
10
0
-10
mmol NH3/g DM
0
3
5
8
12
24
CH
CH+10au
CH+40ac
CH+20au
B)
80
70
60
50
40
30
20
10
0
-10
mmol NH3/g
0
3
8
12
24
incubation time (h)
PL
Pl+10au
Pl+36ac

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **AERTS et al.** Polyphenols and agriculture: beneficial effects of proanthocyanidins in forages. *AGRICULTURE, ECOSYSTEMS & ENVIRONMENT,* 1999, vol. 75 (1-2), 1-12
- **MALCOLM et al.** The effect of four different pasture species compositions on nitrate leaching losses under high N loading. *Soil Use and Management,* 2014, vol. 30 (1), 58-68
- **LUO et al.** Nitrous oxide emission factors for urine and dung from sheep fed either fresh forage rape (Brassica napus L.) or fresh perennial ryegrass (Lolium perenne L.). *ANIMAL,* 2014, vol. 9 (03), 534-543
- **GAWEL et al.** Protein from Lucerne in animals supplement diet. *INTERNATIONAL JOURNAL OF FOOD, AGRICULTURE & ENVIRONMENT, WFL, HELSINKI,* 2014, vol. 12 (2), 314-319
- **DIETZ et al.** Inhibitory effects of Plantago lanceolata L. on soil N mineralization. *PLANT AND SOIL,* 2012, vol. 368 (1-2), 445-458
- **WOODWARD.** Can diverse pasture mixtures reduce nitrogen losses?. *PROCEEDINGS OF THE 5TH AUSTRALASIAN DAIRY SCIENCE SYMPOSIUM : NOVEMBER 13 - 15, 2012, MELBOURNE, AUSTRALIA, AUSTRALASIAN DAIRY SCIENCE SYMPOSIUM ; 5 (MELBOURNE), AU,* 2012, 463-464
- **EDWARDS et al.** Milk production and urination behaviour of dairy cows grazing diverse and simple pastures. *PROCEEDINGS OF THE NEW ZEALAND SOCIETY OF ANIMAL PRODUCTION VOL,* 2015, vol. 75, 79-83
- **JUDSON, H.G. ; MCANULTY, R. ; SEDCOLE, R.** *Proceedings of the New Zealand Grassland Association,* 2009, vol. 71, 201-205
- **BOX, L.A. ; EDWARDS, G.R. ; BRYANT, R.H.** *Proceedings of the New Zealand Society of Animal Production,* 2016, vol. 76, 18-21
- **MOORHEAD, A.J.E. ; PIGGOT, G.J.** *Proceedings of the New Zealand Grassland Association,* 2009, vol. 71, 195-199